# EUROPEAN PATENT APPLICATION

(11) **EP 1 769 803 A1**
(43) Date of publication of application: **04.04.2007**
(21) Application number: 05077118.7
(22) Date of filing: 16.09.2005
(51) Int. Cl.: A61K 38/17, G01N 33/50

(54) **Diagnostics for, medicaments and cosmetics comprising an assembly chaperone for a complex involved in oxidative phosphorylation**

(71) Applicant: Stichting Katholieke Universiteit, 6500 HB Nijmegen (NL)
(72) Inventor: Vogel, Rutger Oscar, 6532 AW Nijmegen (NL); Nijtmans, Leonardus Gerardus Josephus, 6536 BH Nijmegen (NL); Smeitink, Johannes Albertus Maria, 6641 AA Beuningen (NL)
(74) Representative: Winckels, Johannes Hubertus F.

(57) **Abstract**

The invention provides means and methods for diagnosing, typing and treating conditions associated with an oxidative phosphorylation deficiency. The invention further provides assembly chaperones, specifically incorporated in a medicament and in a cosmetic composition, and uses thereof for complexes involved in oxidative phosphorylation. Furthermore the invention provides means and methods for testing compounds for increasing the amount of a complex involved in oxidative phosphorylation.

### Annex to the application documents - subsequently filed sequences listing

SEQUENCE LISTING

<110>  UMC St. Radboud Metabolic and Endocrine Diseases

<120>  Diagnostics for, and medicaments and cosmetics comprising
       assembly chaperone for a complex involved in oxidative
       phosphorylation

<130>  P74533EP00

<140>  EP05077118
<141>  2005-09-16

<160>  10

<170>  PatentIn version 3.3

<210>  1
<211>  19
<212>  RNA
<213>  Artificial Sequence

<220>
<223>  #1 antisense strand primer/probe

<400>  1
acuaacauca ggcuucucc                                                    19


<210>  2
<211>  19
<212>  RNA
<213>  Artificial Sequence

<220>
<223>  #2 antisense strand primer/probe

<400>  2
uaacuauaca ucugauucg                                                    19


<210>  3
<211>  32
<212>  DNA
<213>  Artificial Sequence

<220>
<223>  NDUFAF1forward primer/probe

<400>  3
cgcggaattc atggctttgg ttcacaaatt gc                                     32


<210>  4
<211>  34
<212>  DNA
<213>  Artificial Sequence

<220>
<223>  NDUFAF1reverse primer/probe

<400>  4
cgcgtctaga tttaaaaagc cttgggttaa gctc                                   34


<210>  5
<211>  327
<212>  PRT

<213>  Homo sapiens

<400>  5

<210> 6
<211> 328
<212> PRT
<213> Mus musculus

<400> 6

Met Ser Ser Ile His Lys Leu Leu Thr Gly Ile Tyr Ile His Lys Asn
1               5                   10                  15

Phe Leu Arg Pro Arg Ala Ala Leu Pro Pro Phe Val Gly Ser His Cys
            20                  25                  30

Val Asp Tyr Ser Ser Ser Ser Leu Gln Lys Lys Val Thr Ser Val Asp
        35                  40                  45

Arg Ala Ser Gln Gln Gly Lys Thr Glu Glu Gly Leu Gln Gly His Asp
    50                  55                  60

His Lys Glu Val Ala Leu Asp Ala Pro Ser Pro Asp Arg Thr Pro Glu
65                  70                  75                  80

Val Ser Phe Asp Lys Ala Ile Arg Asp Glu Ala Ile Glu His Phe Arg
                85                  90                  95

Arg Leu Lys Asp Glu Ile Val Ala His Leu Arg Gly Pro Asp Gly Arg
            100                 105                 110

Pro Leu Gln Glu Val Ile Met Glu Gln Ala Arg Val Val Trp Gln Phe
        115                 120                 125

Arg Glu Lys Glu Asp Phe Asp Lys Trp Ile Val Thr Ser Asp Lys Thr
    130                 135                 140

Ile Gly Gly Arg Ser Glu Ile Phe Leu Lys Met Ser Lys Asn Asn Arg
145                 150                 155                 160

Ser Ala Leu Leu Tyr Gly Thr Leu Ser Ser Glu Pro Pro Gln Asp Gly

                165                 170                 175

Asp Ser Arg Gln Ser Gly Tyr Cys Ala Met Ile Ser Arg Ile Pro Arg
            180                 185                 190

Gly Ala Phe Glu Arg Lys Leu Ser Tyr Asp Trp Ser Gln Phe Asn Thr
        195                 200                 205

Leu Tyr Leu Arg Val Arg Gly Asp Gly Arg Pro Trp Met Val Asn Ile
    210                 215                 220

Arg Gln Asp Thr Glu Phe Ile Gln Arg Lys Asn Gln Met Tyr Ser Tyr
225                 230                 235                 240

Phe Met Phe Thr Arg Gly Gly Pro Tyr Trp Gln Glu Val Lys Ile Pro
                245                 250                 255

Phe Ser Lys Phe Phe Phe Ser Asn Gln Gly Arg Val Arg Asp Val Gln
            260                 265                 270

Gly Pro Leu Val Leu Asp Lys Ile Ser Ser Ile Gly Phe Thr Leu Ser
        275                 280                 285

Asp Lys Val Asp Gly Pro Phe Phe Leu Glu Ile Asp Phe Ile Gly Val
    290                 295                 300

Phe Thr Asp Pro Ala His Thr Glu Glu Phe Ala Tyr Glu Asn Ser Pro
305                 310                 315                 320

Val Leu Asn Pro Arg Leu Phe Arg
                325

<210> 7
<211> 340
<212> PRT
<213> Caenorhabditis elegans

<400> 7

Met Ile Thr Ser Phe Pro Val Arg Asn Phe Leu Pro Arg Val Thr Ile
1               5                   10                  15

Thr Ser Cys Ala Phe Phe Ser Lys Glu Ser Thr Ser Ala Ala Gly Thr
            20                  25                  30

Val Ala Glu Lys Lys Glu Ser Glu Leu Gln Lys Phe Ala Gly Lys Ser
        35                  40                  45

Asn Leu Leu Glu Lys Lys Lys Val Gly Ile Phe Gly Thr Ser Thr Val
    50                  55                  60

Pro Ile Asn Thr Asn Phe Pro Asn Pro Lys Gly Val Val Asp Tyr Asp
65                  70                  75                  80

Pro Asp Phe Ser Ile Lys Glu Leu Val Ala Glu Leu Pro Asn Thr Arg
                85                  90                  95

Lys Thr Gln Gly Ala Lys Leu Ser Glu Glu Ile Lys Asn Ala Phe Asn
            100                 105                 110

Ser Val Ser Ile Glu Lys Thr Glu Leu Leu Glu Asp Ile Gly Phe Val
        115                 120                 125

Arg His Asn Glu Ala Arg Val Asp Tyr Arg Phe Asp Thr Gln Glu Lys
    130                 135                 140

Leu Asp Leu Trp Lys Ile Gly Cys Asp Ser Asp Trp Lys Glu Gly Phe
145                 150                 155                 160

Ser Thr Cys Ser Leu Val Asn Ser Asp Arg Gly Ala Thr Val Phe Ser
                165                 170                 175

Gly Asn Ile Ser Thr Lys Val Leu Lys Asp Gly Arg Val Glu Arg Ala
            180                 185                 190

Gly Trp Ala Ser Met Lys Leu Glu Asp Arg Lys Ala Phe Asn Arg Lys
        195                 200                 205

Lys Phe Leu Ser Lys Trp Arg Asn Phe Ser His Leu Leu Leu Lys Val
    210                 215                 220

Arg Gly Asp Gly Arg Ser Tyr Lys Ile Met Leu His Ser Pro Leu Ser
225                 230                 235                 240

Met Asp Phe Thr Trp Gly Asp Ser Phe Ser His Pro Leu His Thr His
                245                 250                 255

Gly Gly Pro Tyr Trp Gln Tyr Glu Lys Ile Pro Phe Ser Lys Phe Phe
            260                 265                 270

His Thr Val Ala Gly Arg Ile Gln Asp Arg Gln Tyr Arg Val Asn Leu
        275                 280                 285

Glu Asp Thr Ser Ser Ile Gly Ile Val Leu Met Asp Arg Ile Asp Gly
    290                 295                 300

Asp Phe Arg Leu Glu Ile Asp Tyr Ile Gly Val Tyr Asn Asp Thr Thr
305                 310                 315                 320

His Val Glu Asp Phe Ala Tyr Glu Thr Tyr Thr Leu Pro Val Phe Asn
                325                 330                 335

Thr His Gly Phe
            340

<210>  8
<211>  296
<212>  PRT
<213>  Drosophila melanogaster

<400>  8

Met Asn Ser Leu Leu Arg Gln Gly Leu Arg Leu Gly Cys Cys Leu Pro
1               5                   10                  15

Ala Val Gln Gln Gln Ile His Thr Thr Ala Val His Arg Thr Phe Trp
            20                  25                  30

Glu Arg Glu Lys Lys Ser Gly Tyr Lys Thr Lys Leu Pro Glu Pro Ser
        35                  40                  45

Lys Lys Gln Met Ile Met Asp Gly Leu Arg Asp Leu Lys Glu Glu Met
    50                  55                  60

Lys Leu Trp Arg Gln Glu Val Lys Glu Gln Phe Glu Ser Asp Pro Ile
65                  70                  75                  80

Leu Val Phe Arg Pro Gly Glu Thr Asp Val Val Phe Asp Phe Lys Ala
                85                  90                  95

Pro Asp Val Leu Asp Lys Trp Thr Val Thr Thr Asp Ala Asp His Gly
            100                 105                 110

Glu Gly Lys Ser Thr Ala Thr Leu Glu Leu Ser Ala Ala Gly Ala Gly
        115                 120                 125

Leu Phe His Gly Gln Val Asn Ser Asp His Thr Lys Asp Gly Ile Ile
    130                 135                 140

Lys Arg Thr Gly Tyr Ala Asn Ile Arg Thr Lys Arg Val Arg Lys Ser
145                 150                 155                 160

Phe Lys Arg Glu Thr Thr Tyr Asp Trp Thr Gln Tyr Asn Met Leu Val
                165                 170                 175

Met Lys Val Arg Gly Asp Gly Arg Ser Tyr Leu Ile Asn Leu His Thr
            180                 185                 190

Glu Gly Tyr Phe Asp Leu Met Trp Asn Asp Ile Tyr His Tyr Val Leu
        195                 200                 205

Tyr Thr Arg Gly Gly Pro His Trp Gln Ile Ala Lys Ile Pro Phe Ser
    210                 215                 220

Lys Phe Phe Leu Ser Ser Lys Gly Arg Val Gln Asp Arg Gln Gly Ala
225                 230                 235                 240

Ile Pro Leu Asn Arg Val Thr His Phe Gly Phe Ser Val Ala Ala Lys
                245                 250                 255

Lys Gly Met Asp Gly Pro Phe Gly Leu Glu Ile Asp Tyr Val Gly Leu
            260                 265                 270

Glu Tyr Asp Pro Ser His Arg Glu Glu Phe Ala Tyr Glu Met Tyr Gln
        275                 280                 285

Thr Pro Lys Tyr Ile Val Ala Thr
    290                 295

<210>  9
<211>  278
<212>  PRT
<213>  Neurospora crassa

<400>  9

Met Arg Ala Thr Gln Ser Leu Pro Phe Lys Ser Phe Trp Ser Arg Ser
1               5                   10                  15

Leu Asp Glu Leu Ser Arg Leu Thr Asn Ile Val Val Lys Ser Glu Asn
            20                  25                  30

Ile Arg Gly Ala Thr Gly Pro Arg Glu Ile His Asn Phe Gln Thr Pro
        35                  40                  45

Glu Ser Val Ala Asp Cys Lys Leu Leu Ser Asp Ala Asp Val Gly Gly
    50                  55                  60

Ser Ser Thr Ala His Leu Asp Trp Val Pro Pro Pro Asn Ala Ile Pro
65                  70                  75                  80

Thr Val Thr Ala Gly Asp Gly Ser Asp Arg Lys Pro Tyr Thr Pro Ile
                85                  90                  95

Pro Gly Ser Tyr Ala Arg Phe His Gly Thr Ile Ser Leu Glu Leu Pro
            100                 105                 110

Thr Asp Arg Arg Glu Ile Ser Arg Thr Gly Tyr Ala Gly Phe Arg Thr
        115                 120                 125

Leu Asp Arg Pro Pro Thr Ile Phe Gly Arg Gly Leu Trp Asp Ile Asp
    130                 135                 140

Pro Tyr Ala Tyr Leu Ala Met Arg Val Lys Thr Asp Ala Arg Ser Tyr
145                 150                 155                 160

Phe Val Asn Val Arg Thr Glu Ser Val Val Pro Leu Asp Leu His Gln
                165                 170                 175

His Arg Leu Phe Val Lys Lys Pro Gly Gln Trp Glu Thr Val Leu Ile
            180                 185                 190

Lys Trp Asn Asp Phe Val Arg Thr Asn His Gly Lys Val Ile Glu Pro
        195                 200                 205

Gln Thr Gly Met Leu Arg Gln Lys Val Leu Ser Ile Gly Phe Ser Thr
    210                 215                 220

Thr Asp Arg Lys Ala Gly Pro Tyr Glu Leu Cys Val Glu Arg Leu Trp
225                 230                 235                 240

Ala Thr Asn Asp Phe Asp Glu Ala Gly Val Val Glu Thr Asp Val Ala
                245                 250                 255

Gly Ala Gln Leu Lys Asn Lys His Gly Glu Lys Val Lys Val Thr Trp
            260                 265                 270

Gly Ala Leu Glu Gln Glu
        275

<210>  10
<211>  158
<212>  PRT
<213>  Aspergillus nidulans

<400>  10

Tyr Arg Leu Tyr Thr Arg His His His Val Arg Asn Ser Glu Ser Ser
1               5                   10                  15

Ser Tyr Asp Pro Leu Ser Pro Tyr Ala Ser Pro Glu Ala Ala Glu Ser
            20                  25                  30

Pro Glu Leu Ala Glu Ala Lys Tyr Pro Thr Gly Ile Pro Pro Ala Leu
        35                  40                  45

Ser Asp Val Pro Pro Pro Ser Thr Ile Met Ser Ser Met Ser Ala Thr
    50                  55                  60

Thr Ser Gly Ser Thr Gly Trp Glu Thr Ile Leu Leu Pro Phe Asn Ser
65                  70                  75                  80

Phe Val Arg Thr Asn His Gly Leu Val Val Glu Pro Gln Thr Ser Ile
                85                  90                  95

Ile Arg Gln Arg Val Lys Ser Val Gly His Trp Leu Thr Asp Arg Val
            100                 105                 110

Glu Gly Pro Tyr Asp Leu Arg Ile His Arg Ile Trp Leu Gln Arg Met
        115                 120                 125

Ser Glu Ala Glu Ile Glu Arg Ala Ser Asn Cys Gly Gln Leu Pro Thr

130                 135                 140

Cys Asp Glu Val Ser Thr Gly Gly Cys Ile Asp Asp Ala Thr
145                 150                 155

## Description

The invention relates to the field human disease. The invention in particular relates to assembly chaperones for complexes involved in oxidative phosphorylation and uses of said chaperones in diagnostics, medicaments and cosmetics.

Mitochondria are essential organelles that constitute the powerhouses of the cell. Defects in these organelles often lead to a variety of severe metabolic disorders affecting the organs that have a high-energy demand, such as muscle and brain. With an incidence of at least 1 in 5000 individuals it is recognized as the most common group of inborn errors of metabolism. Moreover, because programmed cell death (apoptosis) is triggered by mitochondria, defects in these organelles have consequences far beyond the diseases, which brought them initially to our attention and involvement in cancer and neurodegenerative diseases like Alzheimer and Parkinson has been demonstrated. So far no effective treatment is available to cure or improve these disease conditions.

In mitochondria, the high-energy compound ATP is generated by the oxidative phosphorylation (OXPHOS) system, which is located in the inner mitochondrial membrane. The OXPHOS system consists of five multi-subunit enzyme complexes (complexes I to V), of which the assembly is quite intricate. It requires a coordinated expression of both mitochondrial and nuclear encoded subunits, which have to be transported to the mitochondria, where they are inserted into the inner membrane. Together with the addition of prosthetic groups, these subunits are subsequently assembled into enzymatic functional complexes. The nuclear encoded subunits, which mostly contain an import sequence, are translated in the cytosol and imported via the mitochondrial import machinery. The mitochondrial encoded subunits are translated in the mitochondrial matrix. Proper assembly of these complexes requires helper proteins, so called assembly chaperones, which assist, in specific steps of the assembly of the OXPHOS complexes.

The contribution of mitochondrial dysfunction to human disease was already recognised in the late 1980s, when maternally inherited point mutations, as well as deletions arising spontaneously during development, were found to be associated with rare neurological syndromes. Since then, further advances in our understanding have greatly expanded the field of mitochondrial OXPHOS disorders. The mtDNA genotype has been found to be a significant contributor to a range of relatively common, complex or heterogeneous disorders, including diabetes, sensorineural deafness and cardiomyopathy. Many nuclear genes that play roles in mtDNA maintenance or expression have also been found to be involved in disease, including some disorders whose 'mitochondrial basis' was not previously recognized. More controversially, the demonstrable alterations to the OXPHOS system during ageing have given rise to the idea that such defects may play a functionally significant role in the degenerative processes of ageing itself.

Of old, mitochondrial diseases have been correlated with mutations in genes having a function in the mitochondrion. Such genes are located both in the chromosomal- and in the mitochondrial-genome. Mutation of crucial genes negatively affects the function of the mitochondrion and thereby the energy supply of the cell. Although the classical mitochondrial diseases are among the more frequent of the inherited disorders, they are still rare. Recently, it has been recognized that more widely spread diseases such as Parkinson, Alzheimer are associated with deficiencies in the OXPHOS system. Oxidative phosphorylation deficiencies are even associated with the condition of aging.

For instance, in Alzheimer's disease (AD) pathogenesis, increasing evidence implicates mitochondrial dysfunction resulting from molecular defects in oxidative phosphorylation (OXPHOS). Analysis of expression profiles revealed a downregulation of mitochondrial genes in complex I of OXPHOS in both early and definite AD brain specimens. There are some indications that the decrease is more pronounced for complex I relative to the other four complexes. This suggests that in particular complex I is critical for OXPHOS.

Deficiency in one complex may result in over-compensation in other complexes. For instance, a decrease in the amount of complex I may be compensated in increases of complexes III and IV. The presence of such compensation pathways indicates a great demand on energy production in AD patients.

Parkinson's disease has also been associated with a deficiency in oxidative phosphorylation. Although both Parkinson and Alzheimer patients exhibit predominantly brain dysfunction, the OXPHOS system, at least in Parkinson patients is affected systemically. Deficiencies can be, for instance, be found in other organs such as the muscle. In the present invention it was found that complex I deficiency in is involved in premature cell death in the nigrostriatal dopamine pathway, probably due to energy impairment and/or accentuated free radical generation.

Mitochondrial function and, in particular OXPHOS functionality decreases with age. Although, all complexes are involved, most individuals exhibit a decrease in the activity of complex I. Thus also aging is a condition associated with a deficiency of the OXPHOS system. Ageing is a deleterious, progressive, and intrinsic phenomenon in an organism. All individuals, who do not succumb due to accidental death, will be affected in species that age. Abundant evidence implicates a crucial role of mitochondria in this process and the mitochondrial theory of ageing is widely accepted. This theory involves: increased mitochondrial and mitochondrial DNA damage by increased reactive oxygen production (ROS), resulting in progressive respiratory chain dysfunction. Age-related increase in mitochondrial ROS production and mtDNA damage, as well as decrease of respiration and mitochondrial ATP production has been shown in several studies. Strong arguments for the mitochondrial theory of ageing was presented in a recent article, which shows premature aging in mice, which accumulate mitochondrial mutations due to the expression of a proofreading defective mitochondrial DNA polymerase (Trifunovic, et al. Nature 429: 417-423)(for a recent review see, Dufour and Larson Biochim. Biophys. Acta 1658 (2004) 122-132).

In the present invention it was found that over-expression of an assembly chaperone for a complex involved in oxidative phosphorylation (further referred to as assembly chaperone), in a cell leads to an increase in the amount of fully assembled complex. The amount of oxidative phosphorylation complexes (OXPHOS complexes) in a cell is typically a limiting factor in the oxidative phosphorylation, thus increasing the amount of assembled complex leads to increased capacity of the OXPHOS system, thereby increasing the energy supply of cells, particularly in situations of high energy demand.

The present invention therefore provides a method for the treatment of a condition that is associated with an oxidative phosphorylation deficiency in an individual comprising providing cells that are affected by said deficiency with an assembly chaperone for a complex involved in oxidative phosphorylation in a mitochondrion.

Every cell needs energy. Shortage of energy therefore affects the activity of every cell. Thus in principle every cell is affected by a sub-optimal amount of one or more of the OXPHOS complexes. However, the actual amount that is sub-optimal varies from cell to cell. Cells that have a relatively high energy consumption such as brain and muscle cells typically require a higher amount of OXPHOS system complexes than cells that have a low energy consumption, such as resting T-cells. Thus, the cells that are affected by said deficiency associated with an oxidative phosphorylation deficiency are typically, but not necessarily muscle cells or brain cells. Mitochondrial disorders are pleiotropic in their clinical manifestation. Various tissues can be affected like for instance pancreas, heart, liver, eye, inner ear, blood, colon and kidney. In addition, also cells from non-clinically affected tissues like fibroblasts often show a mitochondrial defect.

Cells affected by an OXPHOS deficiency can be treated and provided with a higher amount of OXPHOS complex by providing the cell with an assembly chaperone that is involved with the assembly of said complex. A cell is affected by an OXPHOS deficiency when the OXPHOS capacity is lower than normal (i.e. a comparable cell of the same species from a healthy individual). The capacity is typically lower over a prolonged period of time. Apart from being derived from an individual with an OXPHOS deficiency there are several methods to determine whether a cell has an OXPHOS deficiency, such test encompass but are not limited to oxygen consumption, ATP production capacity, and enzymatic activities of individual OXPOS complexes. (Chretien and Rustin J Inherit Metab Dis. 2003;26(2-3): 189-98). It has surprisingly been found that over-expression of an assembly chaperone of a complex in a cell, results in higher amounts of the fully assembled complex in the cells, (i.e. the mitochondria of the cells). For review of assembly chaperones of the invention, their synthesis, transport and normal function in a cell see Coenen et al. Mitochondrial oxidative phosphorylation system assembly in man: recent achievements. Curr Opin Neurol. 2001 Dec;14(6):777-81; Barrientos et al.Cytochrome oxidase in health and disease. Gene. 2002 Mar 6;286(1):53-63; and Schulte Biogenesis of complex I. J. Bioenerg. Biomembr. 33: 205-212.

In a preferred embodiment said assembly chaperone comprises a chaperone of table 2 or a functional part, derivative and/or orthologue thereof.

Assembly chaperones of table 2, are particularly effective in increasing the amount of the fully assembled complex they are an assembly chaperone for. In case an assembly chaperone is involved with the assembly of more than one complex, for instance but not limited to OXA1, it is preferred that said promiscuous assembly chaperone is used to increase the amount of fully assembled complex I in said cell. In a preferred embodiment said assembly chaperone is of the same species as the cell that is provided with said assembly chaperone. In case of humans it is preferred that said assembly chaperone is a primate assembly chaperone, preferably a human assembly chaperone. In a preferred embodiment said human assembly chaperone comprises
for Complex I: NDUFAF1 (Janssen R, Smeitink J, Smeets R & van den Heuvel L (2002) CIA30 complex I assembly factor: a candidate for human complex I deficiency? Hum Genet 110, 264-270;
   CIA84: (gi:51593777);
   GI:40018641;
   GI:31341340; GI :8895094 (Gabaldon, et al Genes Tracing the evolution of a large protein complex in the eukaryotes, NADH:ubiquinone oxidoreductase (Complex I). J Mol Biol. 2005 May 13;348(4):857-70.) or
   Mimitin (Tsuneoka et al, A novel Myc-target gene, mimitin, that is involved in cell proliferation of esophageal squamous cell carcinoma.J Biol Chem. 2005 May 20;280(20):19977-85)
for Complex III:
   CPB3 (gi:28395057), BCS1 (gi:46397351);
for Complex IV;
   COX11, COX15. COX17, SCO1, SCO2, SURF1, PET191 (Barrientos A, Barros MH, Valnot I, Rotig A, Rustin P, Tzagoloff A Cytochrome oxidase in health and disease. Gene 2002.Mar.6.;286.(1.):53.-63. 286:53-63);
   LRPPRC (Mootha et al. Identification of a gene causing human cytochrome c oxidase deficiency by integrative genomics.Proc Natl Acad Sci U S A. 2003 Jan 21;100(2):605-10.)
   COX19, COX16, PET191 (Tay et al. Studies of COX16, COX19, and PET191 in human cytochrome-c oxidase deficiency. Arch Neurol. 2004 Dec;61(12):1935-7.)
for Complex V :
   ATP11 (gi:17511865), ATP12 (gi:73917623) or for multiple complexes (i.e. promiscuous assembly chaperones):
   OXA1 gi:38372882
   AFG3L, paraplegin, YME1L (Jacobs et al. The mitochondrial inner membrane AAA metalloprotease family in metazoans.FEBS Lett. 2000 Sep 15;481(2):91-5)
   or a human homologue of the following assembly chaperone genes:
for Complex II :
   TCM62 (Dibrov and Lemire The Saccharomyces cerevisiae TCM62 gene encodes a chaperone necessary for the assembly of the mitochondrial succinate dehydrogenase (complex II).J Biol Chem. 1998 Nov 27;273(48):32042-8)
for complex III:
   CBP4 (Sc), CBS2 (Sc)
for complex IV:
   COX18 (Sc), COX14 (Sc), COX20 (Sc), PET197 (Sc)
for complex V :
   ATP10(Sc), FMC1 (Sc)
   or for multiple complexes (promiscuous assembly chaperones):
   MBA1 (Sc)

In a particularly preferred embodiment, said assembly chaperone comprises a NDUFAF1, CIA84, mimitin, CPB3, BCS1, COX10, COX11, COX15, COX17, SCO1, SCO2, SURF1, PET191, LRPPRC, ATP11, ATP12, OXA1 or a functional part, derivative and/or orthologue thereof.

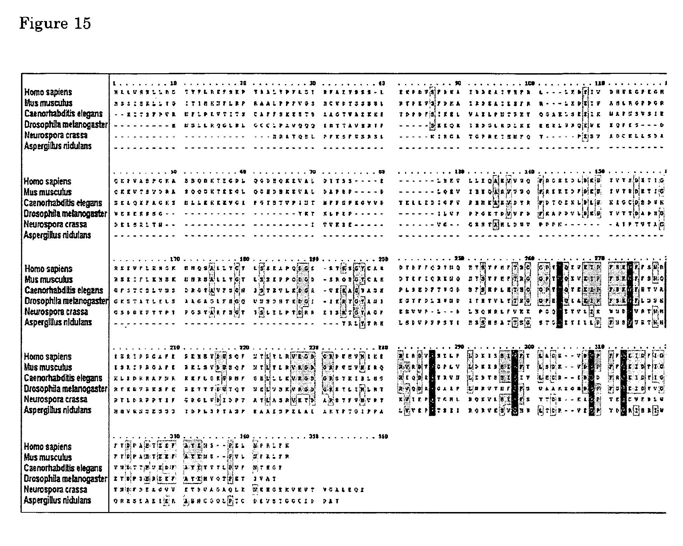

A functional part, derivative and/or orthologue of an assembly chaperone mentioned in the previous paragraphs or of table 2, comprises the same assembly promoting function in kind, not necessarily in amount. Functional domains have been characterized for various assembly chaperones. These are of course part of the invention. A derivatives can be generated for instance by conservative amino acid substitution preferably in area's that exhibit low evolutionary conservation. A sequence comparison for the preferred NDUFAF1 is given in Figure 15. Many different conditions are known to be associated with an oxidative phosphorylation deficiency. In the present invention it is preferred that said condition is a condition listed in table 1. The capacity for oxidative phosphorylation increases by increasing the amount of fully assembled complex in a cell. It is possible to increase said capacity by increasing the amount of fully assembled complexes of any of the complexes I-V. Best results are, however, obtained with increasing said amount of a complex that is particularly associated with said condition. Thus in a preferred embodiment said condition is treated by providing an assembly chaperone that is involved with the assembly of the complex that is associated with said condition, i.e. present in sub-optimal amounts in the mitochondrion. A sub-optimal amount of fully assembled complex can be a lower than normal or required amount of fully assembled complex. It can also be lower activity of the fully assembled complex, for instance, due to a mutation, than normal or required or both. These types of deficiencies can be treated by a method of the invention. In a particularly preferred embodiment said condition comprises Parkinson, Alzheimer or aging, or a combination thereof. Increasing the capacity for oxidative phosphorylation in these conditions increases at least the quality of life of individuals having said condition. It is preferred to treat individuals having said condition at an early stage of the disease. Treatment at an early stage increases the quality of the remaining life at least immediately after treatment. It can also, for instance in the case of Parkinson, delay the progression of the disease. In a preferred embodiment said mitochondrial disease is a Complex I disease. Most mitochondrial disorders are combinations of defects in several OXPHOS complexes. For the present invention, a Complex I disorder is a mitochondrial disorder wherein at least Complex I is affected, preferably due to a reduced amount of fully assembled complex and/or reduced activity of said Complex I. In a preferred embodiment said mitochondrial disordercomprises mitochondrial myopathy, cardiomyopathy, encephalomyopathy, Myoclonic epilepsy, Leigh and Leigh-like syndrome, Leber hereditary optic Neuropathy (LHON), neuropathy ataxia and retinitis pigmentosa (NARP), mitochondrial encephalopathy and stroke-like episodes (MELAS) and myoclonus epilepsy with ragged-red fibers (MERRF)), related diseases (such as Alzheimer's disease, Parkinson's disease, diabetes and deafness) and the process of ageing..

A method of the invention is preferably performed with an assembly chaperone that is involved in the assembly of complex I. Complex I (NADH:ubiquinone oxidoreductase) is the largest multi-protein enzyme of the oxidative phosphorylation system. Increasing the amount of fully assembled complex I increases the capacity for oxidative phosphorylation of the treated cell even when the amount of fully assembled complexes II-V is not increased significantly. This is true also for diseases that are associated with a mutation in one or more of the subunits of complex I.

In a preferred embodiment, said assembly chaperone comprises NDUFAF1 or a functional part, derivative and/or orthologue thereof. Providing said cell with NDUFAF1 increases the amount of fully assembled complex I, thereby increasing the capacity for oxidative phosphorylation of the cell.

NDUFAF1 is a mitochondrial protein. Presently it has been found that it is involved in the complex I assembly process. Reducing the intra-mitochondrial amount of NDUFAF1 by knocking down its expression using, for instance, RNA interference leads to a reduced amount and activity of complex I. NDUFAF1 is associated to two complexes of 600 and 700 kDa in size of which the relative distribution is altered in two complex I deficient patients. Analysis of NDUFAF1 expression in a conditional complex I assembly system shows that the 700 kDa complex may represent a key step in the complex I assembly process. NDUFAF1 is an important protein for the assembly/stability of complex I.

It was further found that increasing the expression of the chaperone NDUFAF1 leads to an increase of the amount of active fully assembled complex I. Thus over-expression of a single protein leads to a specific increase of the human enzyme complex I, consisting of at least 45 different subunits.

The failure to assemble a properly functioning complex I (NADH:ubiquinone oxidoreductase, EC 1.6.5.3) results in complex I deficiency. This is a major contributor to mitochondrial disease and frequently results in early childhood death [1]. Since complex IV (cytochrome c oxidase) deficiency is frequently caused by mutations in an assembly factor [2], it is likely that a similar situation holds for complex I. However, in contrast to the 15 assembly chaperones already found for complex IV, so far no assembly proteins have been described for mammalian complex I. Given the complexity of the enzyme, many assembly factors are likely to be needed and still await detection. We have described a candidate gene, NDUFAF1, which has 28% homology with Neurospora crassa complex I assembly chaperone CIA30 [3].

Two candidate assembly proteins for complex I were found in N. crassa: Complex I Intermediate Associated proteins CIA30 and CIA84 [15].

Knockouts of the cia genes in N. crassa resulted in membrane arm subunit knockout phenotypes. The CIA proteins are thought to chaperone the combination of the small and large membrane arm intermediates of complex I via binding to the large membrane arm intermediate. The binding of CIA84 is transient as the protein cycles between a bound and unbound state. Immunoprecipitations using a CIA84 antibody have resulted in the identification of associated subunits in the membrane arm [15]. In the present invention it was found that NDUFAF1 acts as an assembly protein for complex I in human cells, in line with the proposed function of its homologue CIA30 in the fungus N. crassa. As assembly chaperones, and more in particular mutation thereof, are associated with mitochondrial disease the invention further provides determining in a sample of an individual NDUFAF1 expression

A method for determining whether an individual is suffering from a mitochondrial disease, said method comprising determining in a sample of said individual the expression of NDUFAF1 or determining whether the sequence of NDUFAF1 in said individual differs from a normal allele of said gene or a protein derived there from. When the expression is lower than normal or when NDUFAF1 in said sample is mutated said individual suffers from a mitochondrial disease. In a preferred embodiment said method further comprises classifying said mitochondrial disease as a disease associated with a complex I dysfunction.

In another aspect the invention provides a cosmetic a nucleic acid encoding an assembly chaperone for a complex involved in oxidative phosphorylation in a mitochondrion. In another aspect the invention provides a cosmetic composition comprising an assembly chaperone for a complex involved in oxidative phosphorylation in a mitochondrion. A composition of the invention is particularly suited to revive the skin of a treated individual, particularly in individuals with aged skin, either due to aging or due to excessive exposure to sun. Both conditions are related to the production of free radicals in skin. By increasing the amount of a fully assembled OXPHOS complex in a cell of said individual it is possible to lower the action of free radicals in the skin and at least delay further aging in the skin. As such, one can also use a composition of the invention as a prophylactic, i.e. to at least reduce free radicals that would be capable to act on the skin, if left untreated.

In a preferred embodiment said composition further comprises a vehicle for transporting said nucleic acid and/or said protein into a cell. In case of nucleic acid, it is preferred that said vehicle comprises a liposome or a viral capsid. In case of protein it is preferred that said vehicle comprises a cell penetrating peptide. Several cell penetrating peptides are currently known and many more are expected to be found within a decade. In a preferred embodiment said cell penetrating peptide comprises penetratin, Tat-fragment (48-60), Transportan and amphilic model peptide.

In a preferred embodiment said assembly chaperone comprises NDUFAF1.

In yet another aspect the invention provides a method for analysing an expression profile in a subset of cells of an individual comprising determining in said subset an expression profile of at least one and preferably at least two assembly chaperones for at least one complex involved in oxidative phosphorylation in a mitochondrion. In a preferred embodiment said individual is suffering from or is at risk of suffering from Parkinson, Alzheimer or a mitochondrial disorder. Preferably at least two of said at least two assembly chaperones are assembly chaperones for different complexes. Preferably at least one of said assembly chaperones is an assembly chaperone of table 2 or a functional part, derivative and/or orthologue thereof. In a particularly preferred embodiment said at least one assembly chaperone comprises NDUFAF1. In a preferred embodiment said immunohistochemistry comprises detecting binding of binding body specific for NDUFAF1 peptide 313F-327K, NDUFAF1 peptide 43V-57G or a combination thereof. In a preferred embodiment said amount of said complex is determined by means of electrophoresis and/or immunohistochemistry. Preferably, said electrophoresis comprises native gel-electrophoresis.

In yet another aspect the invention provides a method for testing a compound comprising contacting a cell having a decreased amount of a complex involved in oxidative phosphorylation in a mitochondrion with said compound and determining whether the amount of said complex is increased. With said method it is possible to identified, preferably small, molecules that increase the amount of a fully assembled OXPHOS complex in a cell. Such molecules can be used in a method of the invention for the treatment of a condition that is associated with an oxidative phosphorylation deficiency in an individual. In a preferred embodiment said amount of said complex is determined by means of electrophoresis and/or immunohistochemistry. Preferably, said electrophoresis comprises native gel-electrophoresis. In a preferred embodiment said immunohistochemistry comprises detecting binding of binding body specific for NDUFAF1 peptide 313F-327K, NDUFAF1 peptide 43V-57G or a combination thereof.

A binding body specific for NDUFAF1 peptide 313F-327K, NDUFAF1 peptide 43V-57G preferably comprises an antibody or a functional part, derivative and/or analogue of said antibody. Antibodies are well known in the art and need no further explanation here. A functional part, derivative and/or analogue of an antibody of the invention comprises the same peptide binding function in kind, not necessarily in amount. A part of an antibody of the invention comprises at least the peptide binding part. Non-limiting parts are FAB-fragments or single chain Fv fragments. It has been found that the binding property of an antibody is contained in a very small region consisting of a variable part (the CDRs) that provide the binding function and a structural part (the framework) that provides the structural context of the binding body. Monobodies comprising a specificity for a peptide as mentioned above, are therefore also suitable part of an antibody of the invention. A derivative comprises at least one, preferably, conservative amino acid substitution, preferably in a region not involved in peptide recognition, or the framework region. In a preferred embodiment said antibody is a poly-clonal antibody. Thus the invention further provides a binding body specific for NDUFAF1 peptide 313F-327K, NDUFAF1 peptide 43V-57G or a combination thereof.

The invention further provides a method for increasing the amount of a complex involved in oxidative phosphorylation in a mitochondrion, comprising providing a cell comprising said mitochondrion with an assembly chaperone for said complex. In a preferred embodiment said complex is complex I. Preferably, said assembly chaperone comprises NDUFAF1 or a functional part, derivative and/or orthologue thereof.

In another embodiment the invention provides the use of an assembly chaperone for a complex involved in oxidative phosphorylation in a mitochondrion for the preparation of a medicament for the treatment of a condition that is associated with an oxidative phosphorylation deficiency of cells. The invention further provides use of an assembly chaperone for a complex involved in oxidative phosphorylation in a mitochondrion for increasing the amount of said complex in a mitochondrion of a cell.

Assembly of OXPHOS complexes is intricate and involves various intermediates of which the production is often coordinated in a cell. In mitochondrial disease, assembly of said complexes is often different from normal. In the presence invention, this difference is exploited in an assay that discriminates intermediates of fully assembled complex. Said intermediates may differ in amount in mitochondrial disease. However, typically different intermediates are detected in mitochondrial disease. Mitochondrial disease and/or the stage thereof (starting, intermediate or full blown) can be typed by scrutinizing intermediates of assembly of said complex. Parameters that are relevant for such typing are preferably: which intermediates are formed, how many different ones are present, how much "normal" ones are not present and how much of a "normal" and/or a different intermediate is present. In a preferred embodiment said typing comprises generating a pattern of assembly intermediates in a separation process and comparing said generated pattern with a reference, such as a healthy control. In a further aspect the invention therefore provides a method for typing a disease comprising separating complex assembly intermediates in a sample of said individual suffering from said disease and determining relative amounts of two or more of said intermediates and/or altered separation properties of one or more of said complex assembly intermediates. Separation of assembly intermediates is preferably performed under non-denaturing conditions. In this way intermediates remain as they are. Upon said separation, further separation steps may be performed which may be both non-denaturing or denaturing. In a preferred embodiment said two or more intermediates are visualized by visualizing the presence in said intermediates of an assembly chaperone. Preferably, said visualized assembly chaperone comprises NDUFAF1

The invention further provides a method for typing a disease that affects an individual comprising separating complex assembly intermediates in a sample of said individual and determining relative amounts of two or more of said intermediates and/or altered separation properties of one or more of said complex assembly intermediates. In a preferred embodiment said complex intermediates are complex I intermediates. Preferably, said complex I intermediates are detected by means of a binding body specific for NDUFAF1.

Complex I is the first of five multi-protein complexes, which together constitute the oxidative phosphorylation system. In this system NADH is oxidised by complex I, after which electrons are transferred via electron carriers (ubiquinone and cytochrome c) and via complexes III and IV to the final electron acceptor molecular oxygen. The energy of this transfer is used to translocate protons across the mitochondrial inner membrane. The thus generated proton gradient is used by complex V (ATP synthase) to generate ATP.

Mammalian complex I is an L-shaped structure consisting of at least 45 subunits [4,5,6] of which seven are encoded by the mitochondrial genome. The complex can be subdivided into three functionally distinct fragments. The NADH dehydrogenase segment includes the redox cofactor flavomononucleotide, which is involved in the oxidation of the NADH substrate. The hydrogenase part contains several iron sulphur clusters, which are involved in electron transfer to the electron transporter ubiquinone. The membrane bound transporter part of complex I is involved in proton translocation. Whether and how electron transport and proton translocation are coupled is yet uncertain [7].

Assembly of complex I is an intricate process, which has been studied in several organisms [8,9,10]. The most extended investigation of complex I assembly was performed for the fungus Neurospora crassa [11,12]. In the proposed model, the hydrophilic peripheral arm and the hydrophobic membrane arm of complex I are assembled independently before being joined together. The search for a more detailed description of the assembly pathway has recently resulted in the publication of the first models for human complex I assembly [13,14]. Both models differ considerably, illustrating the fact that the complex I assembly pathway is far from solved. The model we describe shows considerable homology to N. crassa complex I assembly. We propose that, analogous to the situation in N. crassa, complex I is assembled semi-sequentially: discrete functional modules are assembled independently and are joined in several steps to form a peripheral arm and a membrane arm assembly intermediate. In more detail, this entails the formation of perhipheral arm assembly intermediate D (600 kDa) from intermediates H (80 kDa), G (150 kDa) and F (250 kDa) and the formation of membrane arm assembly intermediate C (700 kDa) from the combination of membrane proteins with intermediate E (400 kDa). Peripheral arm intermediate D consists of a core or highly conserved hydrophilic subunits (such as the 49 kDa, 39 kDa and 30 kDa subunits), while membrane arm intermediate C consists of highly conserved hydrophobic subunits (such as ND1 and ND6). These two key intermediates are combined to form assembly intermediate B (950 kDa) and finally holo-complex I (A, 1 MDa) [14].

It has been found that alterations in the pattern and/or amounts of respective assembly intermediates, particularly for complex I is indicative for the absence or presence and for the type of complex I related OXPHOS deficiency.

### Examples

### Example 1

### Materials and Methods

### Generating NDUFAF1 antibody

To produce antibodies against NDUFAF1, two oligopeptides were selected. The first peptide corresponds to the mid-portion of the NDUFAF1 sequence, amino acids 43V-57G; the second peptide corresponds to the C-terminus of the protein, amino acids 314F-327K. A mixture of both peptides was coupled to keyhole limpet hemocyanin, serving as an immunogenic carrier. Rabbits were immunised with an injection of the antigen-carrier conjugate, followed by three subsequent boosters, one every three weeks. The two antisera were collected from a final bleeding and tested for specific detection of the NDUFAF1 protein by Western blotting. Both antisera displayed comparable specificity.

### Cell cultures

JM109 E.coli cells (Promega) were cultured in Luria Burtani (LB) medium and the appropriate antibiotic was added to the medium. 143B206 ρ0 cells were cultured in Dulbecco's Modified Eagle Medium (DMEM) (Bio Whitaker) supplemented with 5% fetal calf serum (FCS), antibiotics, 1 mM uridine and 100 µg/ml bromodeoxyuridine. HeLa cells and 143B osteosarcoma cells were cultured in DMEM supplemented with 10% FCS and antibiotics. HEK293 T-RExtm cells without the TO/NDUFAF1 construct were cultured in the same medium to which 5 µg/ml blasticidin (Invitrogen) was added to maintain the repressor construct. To HEK293 T-RExtm cells containing the TO/NDUFAF1 construct additionally 300 µg/ml Zeocintm (Invitrogen) was added to maintain the inducible construct.

### siRNA transfection

For transfection, HeLa cells were plated in DMEM supplemented with 10% FCS (without antibiotics) in 24-well plates with a cell density of 1.5 x 104 cells per well. The next day, cells were transfected with siRNA duplex (control: Cyclophilin B (Dharmacon), NDUFAF1: #1 antisense strand: 5'-ACUAACAUCAGGCUUCUCC dTdT -3', #2 antisense strand: 5'-UAACUAUACAUCUGAUUCG dTdT -3') in 3 µl oligofectamine (Invitrogen) to achieve a final concentration of 10 nM siRNA per well. Cells were incubated at 37°C in a CO2 incubator for 48-72 hours until they were ready to assay for gene knockdown or to perform a second transfection.

### Cellular fractionation

Approximately 5 x 106 HEK293 cells were harvested, washed in PBS, resuspended in an appropriate isotonic buffer (0.25 M sucrose, 5 mM Tris-HCl, pH 7.5, and 0.1 mM PMSF) and homogenised using a glass teflon homogeniser. Unbroken cells and nuclei were pelleted by centrifugation at 600 g for 15 minutes. Supernatants were centrifuged at 10,000 g for 25 minutes. The resulting supernatant was isolated as the cytoplasmic fraction and the mitochondrial pellet was washed once with the isotonic buffer containing 1 mM EDTA, pH 8.

### Creating the inducible NDUFAF1 construct and transfection into HEK293 T-RExtm cells

A PCR of total cell cDNA was performed to specifically amplify NDUFAF1 cDNA using Native Pfu DNA polymerase (Stratagene). Primer sequences are (5' - 3'):
NDUFAF1forward: CGCGGAATTCATGGCTTTGGTTCACAAATTGC
NDUFAF1reverse: CGCGTCTAGATTTAAAAAGCCTTGGGTTAAGCTC

The amplified fragment and pcDNA4/TO/myc-His A (Invitrogen) (TO) were digested with EcoRI and XbaI restriction enzymes (Gibco) and subsequently ligated using T4 DNA Ligase (Invitrogen) and transformed into JM109 competent E. coli cells (Promega). Clones containing the plasmid were obtained by kanamycin (Sigma Aldrich) selection and were cultured to obtain sufficient construct for transfection. After sequence verification, transfection of the construct was performed on HEK293 T-RExtm cells (Invitrogen), which stably repress the tetracycline repressor operon, using the Superfect® Transfection Reagent (Qiagen). Cells were grown to a confluency of 60-70% in a 6-well plate and cells in each well were transfected by adding 2 µg of the construct according to the protocols described in the Superfect Transfection Reagent Handbook (Qiagen). Stable clones were obtained by culturing under selective pressure of 300 µg/ml Zeocintm (Invitrogen). HEK293 T-RExtm clones containing the TO/NDUFAF1 construct were induced for expression of the transgene by adding 0,1-1 µg/ml doxycycline to the growth medium for the times indicated in the results section.

### Immunofluorescence assay

A culture of a TO/NDUFAF1 containing HEK293 T-RExtm clone was seeded onto a coverslip in a 24-wells plate and grown overnight to achieve a confluency of 50% on the day of the assay. Cells were prestained with Mitotracker Red (Molecular Probes) prior to paraformaldehyde fixation. For fluorescence imaging, cell preparates were attached to the stage of an inverted microscope (Axiovert 200 M, Carl Zeiss, Jena, Germany) equipped with a x63 Plan NeoFluar (NA 1.25) objective (Carl Zeiss). Alexa Fluor®@ 488 and Mitotracker Red were excited at 488 nm and 570 nm respectively, using a monochromator (Polychrome IV, TILL Photonics, Gräfelfing, Germany). Fluorescence emission light was either directed by a 505DRLPXR dichroic mirror (Omega Optical Inc., Brattleboro, USA) through a 535AF26 emission filter (Alexa Fluor® 488) or by a 595DRLP dichroic mirror through a 645AF75 emission filter (Mitotracker Red) onto a CoolSNAP HQ monochrome CCD-camera (Roper Scientific, Vianen, The Netherlands). All hardware was controlled with Metafluor 6.0 software (Universal Imaging Corporation, Downingtown, USA) running on a PC equipped with 1 Gb RAM running Windows XP Professional.

### Digitonin isolation and solubilisation of mitochondria

Approximately 1 x 106 trypsin-harvested cells were pelleted and resuspended in 100 µl of cold PBS to which 100 µl of 4% digitonin (w/v) was added to achieve a final concentration of 2% digitonin. This sample was shortly vortexed and incubated on ice for 10 minutes to solubilize cell membranes. After this, 1 ml of cold PBS was added and the sample was centrifuged at 10000 g for 10 minutes at 4°C to obtain a mitochondria-enriched organelle pellet. To remove traces of digitonin, this pellet was washed twice with 1 ml of cold PBS. Mitochondrial proteins were solubilised by the addition of 100 µl of ACBT (1.5 M aminocaproic acid, 75 mM Bis-Tris pH 7.0) and 20 µl of 10% n-dodecyl β-D-maltoside (w/v) and incubation on ice for 10 minutes. The solubilised proteins are retained in the supernatant after centrifugation at 10000 g for 25 minutes at 4°C, which was used for further analysis.

### Gel electrophoresis and in-gel activity assays

The protein concentration for BN-PAGE and SDS-PAGE was determined in the n-dodecyl β-D-maltoside solubilised supernatants before adding coomassie blue containing sample buffer, using a MicroBCA protein assay kit (Pierce). Blue-native 5-15% gradient gels were loaded with 40 µg of digitonin-isolated mitochondria and, after electrophoresis, were further processed for Western blotting, second dimension 10% SDS-PAGE or in-gel activity assays as described earlier [18]. SDS-PAGE analysis was performed by loading 40 µg of protein/lane on 10% SDS-PAGE gels as described before [14].

### Blotting, detection

Blue-native and SDS gels were blotted onto PROTRAN Nitrocellulose Transfer Membrane (Schleicher and Schuell BioScience) according to standard procedures. Primary antibodies used are against the following complex I subunits: NDUFA3 (30 kDa), NDUFA9 (39 kDa), NDUFB6 (B17) (Molecular Probes), ND1 (a gift from Dr. A. Lombes, France), ND6 (a gift from Professor R. Capaldi, USA), Complex II 70 kDa subunit (Molecular Probes), CoreII (Molecular Probes), NDUFS5 (15 kDa) (Molecular Probes), HSP70 (Abcam), c-myc (Invitrogen) and NDUFAF1. Secondary antibodies used are Swine Anti-Rabbit PerOxidase (SWARPO), Goat Anti-Rabbit PerOxidase (GARPO) and Goat Anti-Mouse PerOxidase (GAMPO) (Molecular Probes). Signal detection was performed using the ECL® plus Western Blotting Detection System (Amersham Biosciences). After ECL, the blots were exposed to X-OMAT UV film (KODAK). ECL signals were quantified using the ImagePro-Plus 4.1 image analysis software (Media Cybernetics, Silver Spring, MD, USA).

### Results

### NDUFAF1 localises in the mitochondrion

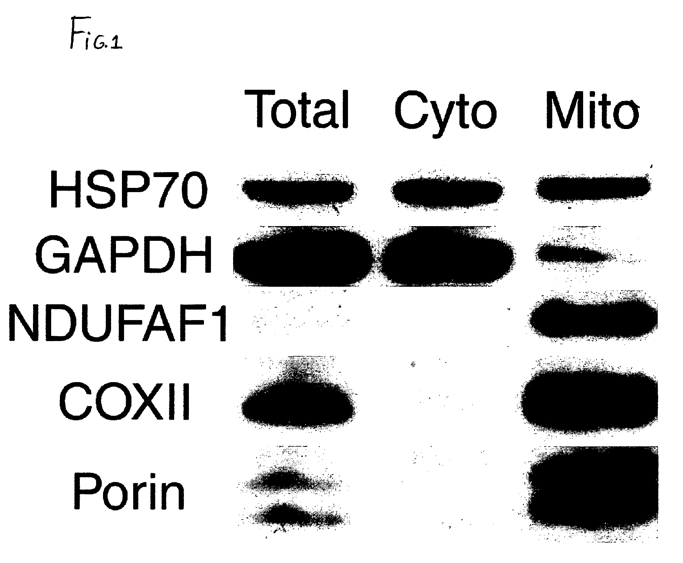

To analyse the subcellular localisation of NDUFAF1 we harvested HEK293 cells at 90% confluency and prepared protein samples of total cell lysate, isolated mitochondria and the cytoplasmic fraction. A Western blot of this gel was incubated with antibodies against NDUFAF1, a cytoplasmic marker (GAPDH), a marker for the mitochondrial inner membrane (COXII), a marker for the mitochondrial outer membrane (porin) and a marker for both cytoplasm and the mitochondrial matrix (HSP70) (Fig. 1). NDUFAF1 is clearly more abundant in isolated mitochondria than in the total cell protein extract and is absent in the cytoplasmic protein extract. These data show a specific localisation of NDUFAF1 in the mitochondrion.

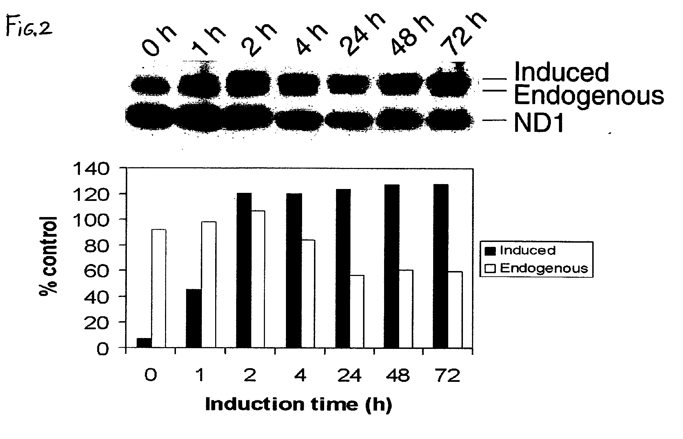

To confirm this result and to use another antibody for localisation studies, we decided to create a construct in which the NDUFAF1 gene was tagged with an immunogenic epitope. We cloned NDUFAF1 into an inducible vector in-frame with a c-myc and 6xHis tag and transfected this construct into a human embryonic kidney cell line (HEK293), which stably expresses the tetracycline repressor gene. Stable clones were selected and induction of protein expression was tested using Western blot analysis. Digitonin-isolated mitochondria were obtained from cultures of an inducible clone after 0, 1, 2, 4, 24, 48 and 72 hours of induction with 0.1 µg/ml of doxycycline and analysed by Western blotting (Fig. 2). This concentration of doxycyline does not interfere with mitochondrial protein translation [14]. Using both the NDUFAF1 and the c-myc antibodies both the endogenous and the induced (slightly larger than the endogenous protein due to the C-terminal tags) NDUFAF1 could be observed after induction.

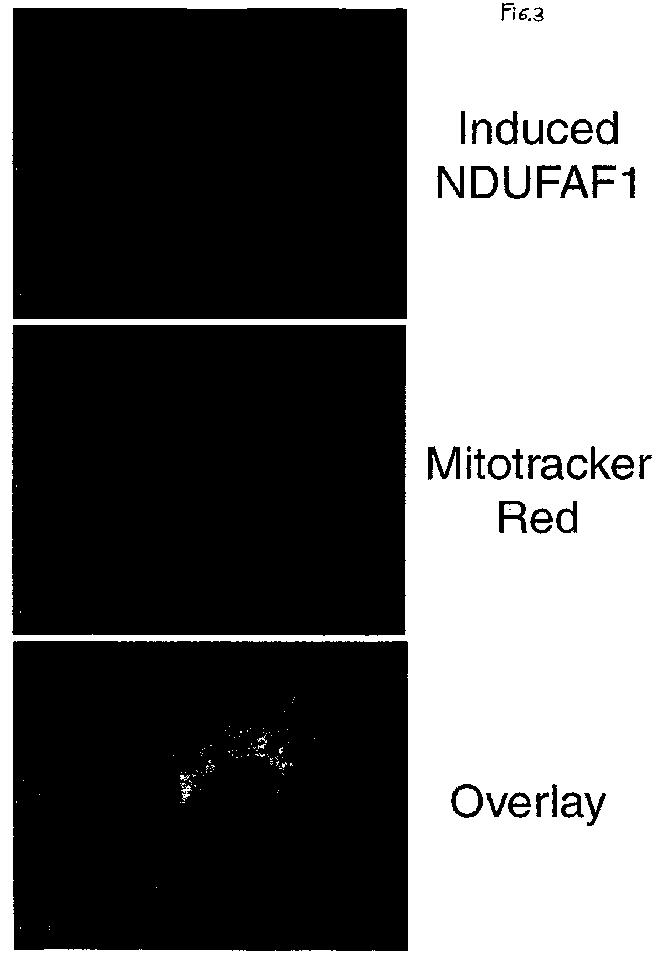

To investigate the cellular localisation of the NDUFAF1-c-myc fusion protein we used a monoclonal c-myc antibody in a immunohistochemical experiment, further incubated with a secondary antibody to which a fluorescent Alexa probe was coupled. The overlay of the Alexa dye staining with mitochondrial control Mitotracker Red shows that induced NDUFAF1 indeed migrates to the mitochondrion (Fig. 3). Biochemical fractionation of the NDUFAF1-c-myc expressing cells indicates that this fusion protein also exclusively localises in mitochondria (results not shown).

### NDUFAF1 knock down reduces the complex I level and activity

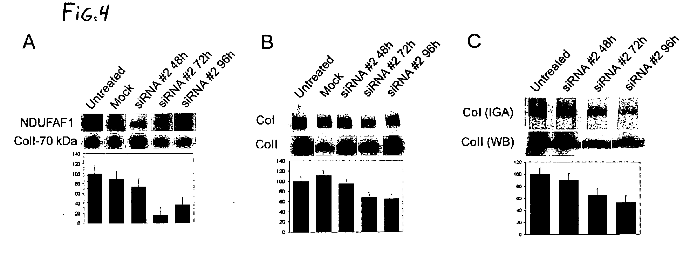

Based on its homology with alleged complex I chaperone CIA30 in N. crassa, NDUFAF1 is an interesting candidate assembly protein for human complex I. The possible involvement of NDUFAF1 in complex I assembly/stability was investigated by performing RNA interference experiments. To knock down NDUFAF1 protein expression, two different small interfering RNA (siRNA) oligonucleotides were designed for targeting NDUFAF1 mRNA. Both siRNA's displayed similar knock down effects. Fig. 4 shows a transfection of 48 hours, followed by consecutive transfections of 48, 72 or 96 hours with 10 nM of siRNA #2 (data for siRNA #1 not shown). RNA interference effects are analysed for NDUFAF1 protein (Fig. 4A), fully assembled complex I (Fig. 4B) and complex I in-gel activity (Fig. 4C). NDUFAF1 expression can be knocked down to less than 30% of the control signal (Fig. 4A, lanes 4 and 5). This leads to a 40% decrease of fully assembled complex I (Fig. 4B, lane 5). Complex I activity is compromised as well, as can be seen by the 50% decrease of signal in Fig. 4C (lane 5). Interestingly, longer second incubations with siRNA lead to a greater inhibitory effect. Control transfections with a siRNA targeting cyclophilin B performed under the same circumstances did not lead to a reduction of the NDUFAF1 signal, nor to a reduction in the amount of fully assembled complex I (data not shown). This analysis shows that knockdown of NDUFAF1 protein leads to a decrease in the amount and activity of fully assembled complex I. Conversely, preliminary evidence suggests that overexpression of NDUFAF1 leads to an increase in the expression of complex I (data not shown).

### NDUFAF1 is present in two high molecular weight protein complexes

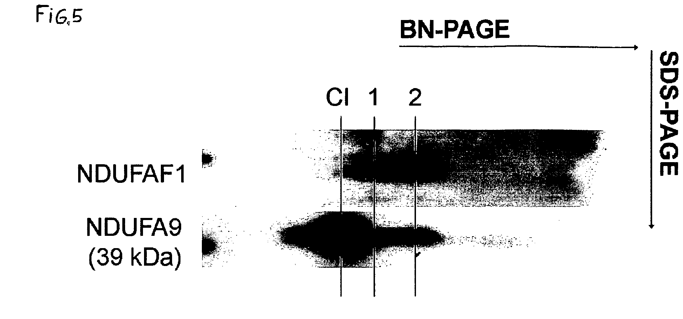

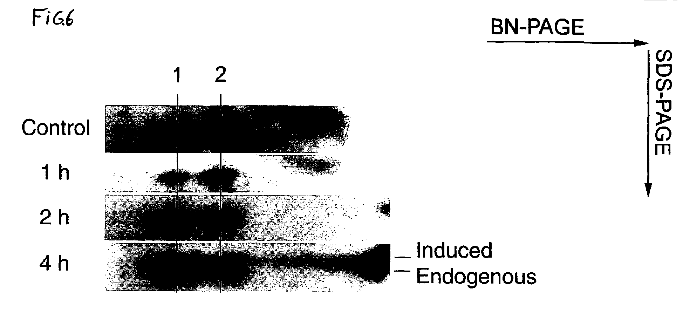

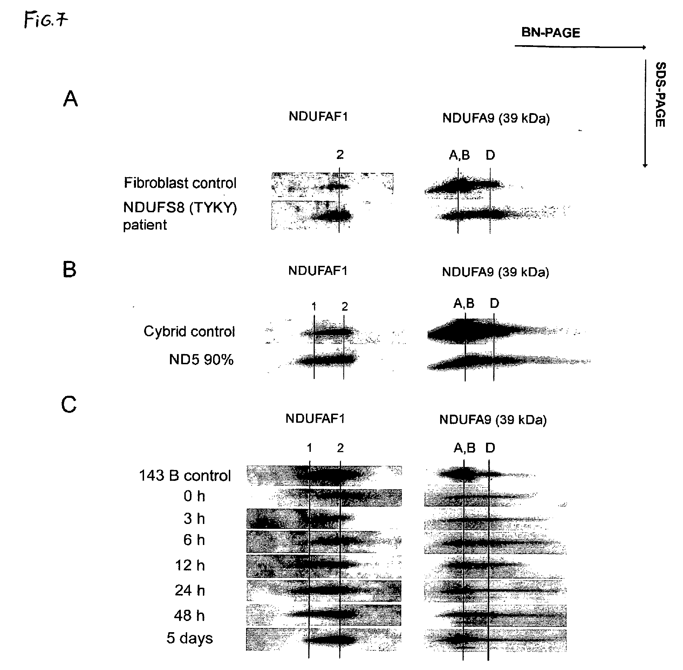

To investigate whether NDUFAF1 is present in high molecular weight protein complexes, its expression pattern was analysed on Western blots of two-dimensional blue-native/SDS gels (2D BN/SDS-PAGE). Control HEK293 cells were harvested at 90% confluency and lysates of digitonin-isolated mitochondria were run on 2D BN/SDS-PAGE gels before blotting and antibody detection (Fig. 5). Two NDUFAF1-containing high molecular weight complexes of about 600 and 700 kDa can be observed (Fig. 5, complexes 2 and 1, respectively). As a size reference, complex I (1 MDa) is shown by using an antibody against the NDUFA9 (39 kDa) subunit of complex I. The expression pattern of NDUFAF1 when it is overexpressed was analyzed by using the doxycycline inducible expression system in combination with 2D BN/SDS-PAGE gels. After 4 hours of induction (Fig. 6), induced NDUFAF1 can be seen to migrate from its monomeric form towards the complexes of 600 and 700 kDa, confirming the data observed for endogenous NDUFAF1 in the control situation.

### NDUFAF1 expression pattern changes in patients with mutations leading to complex I assembly defects

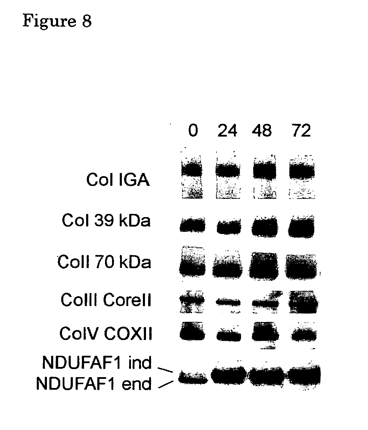

Our complex I assembly model [14] confirms the separate assembly of membrane and peripheral arms described in previous assembly models in N. crassa. A clue about the stage at which NDUFAF1 may operate in this human model can be obtained by screening for the NDUFAF1 expression pattern in complex I deficient patients. For this study, we have used fibroblasts from a NDUFS8 (TYKY) patient (manuscript in preparation) and a ND5 patient cybrid cell line with 90% of heteroplasmy (D393G, see reference 19) as representatives of both a peripheral arm and a membrane arm subunit mutation. Cells were harvested to obtain mitochondrial lysates, which were run on 2D BN/SDS-PAGE gels for Western blotting and antibody incubation (Fig. 7). The NDUFS8 (TYKY) patient cell line is represented in Fig. 7A and the ND5 cybrid cell line is represented in Fig. 7B.

For both patients, complex I expression analysis using NDUFA9 (39 kDa) antibody reveals a decrease in the amount of holo-complex I (represented by A, see reference 14 for detailed description of the composition of complex I assembly intermediates). Less NDUFAF1 is observed in the control fibroblast cell line used for the NDUFS8 (TYKY) patient compared to the cybrid control cell line, and the 700 kDa complex seems absent (Fig. 7A, NDUFAF1 panels). This difference in expression intensity is observed more often and appears to be cell type dependent [14]. Irrespective of this, what can clearly be seen is the strong increase of NDUFAF1 in the 600 kDa complex in the patient compared to the control. So, interestingly, complex I and its assembly intermediates are less abundant whereas more NDUFAF1 is present in the 600 kDa complex.

Additionally, the ND5 patient displays a different relative distribution of NDUFAF1 between the 600 and 700 kDa complexes compared to the control (Fig. 7A, complexes 2 and 1). NDUFAF1 is more prominently present at 700 kDa in the 90% cybrid compared to the control. Since the observed effects are the consequence of complex I membrane arm subunit mutation, this allows the possibility that NDUFAF1 is involved in membrane arm assembly.

### NDUFAF1 expression pattern changes in a conditional complex I assembly system

To further investigate this, we used a conditional complex I assembly system. A high concentration (10 µg/ml or more) of doxycycline results in the inhibition of mitochondrial protein synthesis. Releasing this inhibition allows investigation of the complex I assembly process and has recently been used in our group to establish a human complex I assembly model [14]. Using this system in combination with 2D BN/SDS-PAGE analysis of NDUFAF1 expression allows investigation of the possible involvement of NDUFAF1 with complex I membrane arm assembly intermediates (Fig. 7C). The ratio of the 600 and 700 kDa complexes (Fig. 7C, complexes 2 and 1 respectively), shows a remarkable change after the resumption of mitochondrial protein synthesis. In control 143B osteosarcoma cells, NDUFAF1 is predominantly present in the 600 kDa complex (Fig. 7C, complex 2). After five days of inhibition of mitochondrial protein synthesis (Fig. 7C, t = 0 hours), a minor amount of NDUFAF1 is still present in this complex. At 12 hours after release of inhibition, NDUFAF1 becomes more predominant in the 700 kDa complex (Fig. 7C, complex I) up to 48 hours after release of inhibition. After this time, the amount of NDUFAF1 in the 700 kDa complex decreases and finally returns to the wild-type state in the 600 kDa complex after five days. It seems that NDUFAF1 appears in the 700 kDa complex while complex I assembly proceeds and releases when the control complex I amount is assembled (Fig. 7C, complex A). These kinetics are not displayed for the 600 kDa NDUFAF1 complex, which is still present after 5 days of doxycycline inhibition and increases gradually while complex I assembly proceeds (Fig. 7C, complex 2). The expression kinetics described above support the notion that when complex I membrane arm assembly is either disturbed or induced, NDUFAF1 becomes more abundant in the 700 kDa complex.

### Example 2

### Experiment: overexpression of NDUFAF1 increases mitochondrial complex I

We created a cell-line in which we could induce expression of the chaperone NDUFAF1 by adding the component doxycycline. The expression of NDUFAF1 was checked by werstern blotting using a NDUFAF1 specific antibody. The expression of fully assembled complex I was determined using a native gel system in combination with western blot detection using complex I specific antibodies. Complex I activity was determined using an Complex I specific activity assay.

Analysis of amount and activity of complex I revealed that 48 and 72 hours after a 4 hours induction pulse of NDUFAF1, complex I was increased 4-8 fold (see Figure 1). In parallel, complex III of the respiratory chain was increased albeit at a lesser extent. Complexes II and IV remained constant. This result has been verified by several repetitions of the experiment.

### Generating NDUFAF1 antibody

To produce antibodies against NDUFAF1, two oligopeptides were selected. The first peptide corresponds to the mid-portion of the NDUFAF1 sequence, amino acids 43V-57G; the second peptide corresponds to the C-terminus of the protein, amino acids 314F-327K. A mixture of both peptides was coupled to keyhole limpet hemocyanin, serving as an immunogenic carrier. Rabbits were immunised with an injection of the antigen-carrier conjugate, followed by three subsequent boosters, one every three weeks. The two antisera were collected from a final bleeding and tested for specific detection of the NDUFAF1 protein by Western blotting. Both antisera displayed comparable specificity.

### Creating the inducible NDUFAF1 construct and transfection into HEK293 T-RExtm cells

A PCR of total cell cDNA was performed to specifically amplify NDUFAF1 cDNA using Native Pfu DNA polymerase (Stratagene). Primer sequences are (5' - 3'):
NDUFAF1forward: CGCGGAATTCATGGCTTTGGTTCACAAATTGC
NDUFAF1reverse: CGCGTCTAGATTTAAAAAGCCTTGGGTTAAGCTC

The amplified fragment and pcDNA4/TO/myc-His A (Invitrogen) (TO) were digested with EcoRI and XbaI restriction enzymes (Gibco) and subsequently ligated using T4 DNA Ligase (Invitrogen) and transformed into JM109 competent E. coli cells (Promega). Clones containing the plasmid were obtained by kanamycin (Sigma Aldrich) selection and were cultured to obtain sufficient construct for transfection. After sequence verification, transfection of the construct was performed on HEK293 T-RExtm cells (Invitrogen), which stably repress the tetracycline repressor operon, using the Superfect® Transfection Reagent (Qiagen). Cells were grown to a confluency of 60-70% in a 6-well plate and cells in each well were transfected by adding 2 µg of the construct according to the protocols described in the Superfect Transfection Reagent Handbook (Qiagen). Stable clones were obtained by culturing under selective pressure of 300 µg/ml Zeocintm (Invitrogen). HEK293 T-RExtm clones containing the TO/NDUFAF1 construct were induced for expression of the transgene by adding 0,1-1 µg/ml doxycycline to the growth medium for the times indicated in the results section.

### Digitonin isolation and solubilisation of mitochondria

Approximately 1 x 106 trypsin-harvested cells were pelleted and resuspended in 100 µl of cold PBS to which 100 µl of 4% digitonin (w/v) was added to achieve a final concentration of 2% digitonin. This sample was shortly vortexed and incubated on ice for 10 minutes to solubilize cell membranes. After this, 1 ml of cold PBS was added and the sample was centrifuged at 10000 g for 10 minutes at 4°C to obtain a mitochondria-enriched organelle pellet. To remove traces of digitonin, this pellet was washed twice with 1 ml of cold PBS.

Mitochondrial proteins were solubilised by the addition of 100 µl of AC/BT (1.5 M aminocaproic acid, 75 mM Bis-Tris pH 7.0) and 20 µl of 10% n-dodecyl β-D-maltoside (w/v) and incubation on ice for 10 minutes. The solubilised proteins are retained in the supernatant after centrifugation at 10000 g for 25 minutes at 4°C, which was used for further analysis.

### Gel electrophoresis and in-gel activity assays

The protein concentration for BN-PAGE and SDS-PAGE was determined in the n-dodecyl β-D-maltoside solubilised supernatants before adding coomassie blue containing sample buffer, using a MicroBCA protein assay kit (Pierce). Blue-native 5-15% gradient gels were loaded with 40 µg of digitonin-isolated mitochondria and, after electrophoresis, were further processed for Western blotting, second dimension 10% SDS-PAGE or in-gel activity assays as described earlier [18]. SDS-PAGE analysis was performed by loading 40 µg of protein/lane on 10% SDS-PAGE gels as described before [14].

### Blotting, detection

Blue-native and SDS gels were blotted onto PROTRAN Nitrocellulose Transfer Membrane (Schleicher and Schuell BioScience) according to standard procedures. Primary antibodies used are against the following complex I subunits: NDUFA9 (39 kDa) (Molecular Probes), Complex II 70 kDa subunit (Molecular Probes), CoreII (Molecular Probes), COXII (Molecular Probes), c-myc (Invitrogen) and NDUFAF1. Secondary antibodies used are Goat Anti-Rabbit PerOxidase (GARPO) and Goat Anti-Mouse PerOxidase (GAMPO) (Molecular Probes). Signal detection was performed using the ECL® plus Western Blotting Detection System (Amersham Biosciences). After ECL, the blots were exposed to X-OMAT UV film (KODAK). ECL signals were quantified using the ImagePro-Plus 4.1 image analysis software (Media Cybernetics, Silver Spring, MD, USA).

Proof of principle: adding NDUFAF1 to a complex I deficient cell line can alleviate the complex I deficiency

Expression of the complex I specific chaperone NDUFAF1 will be increased in control fibroblasts and in five patients derived fibroblast cell lines with a decreased amount of fully assembled complex I, which are available in our centre.

Because primary human skin fibroblasts are refractory to most conventional transfection protocols, we use a baculoviral system to express NDUFAF1 and in these cells. The latter system, which is normally used for protein production in Spodoptera frugiperda 9 insect cells, was made suitable for protein expression in mammalian cells by first removing the herpes simplex virus thymidine kinase polyadenylation signal from the pFastBacTMDual vector (Invitrogen) using AccI and XhoI, by next, after blunting and ligation, removing both the p 10 and polyhedron promoter with SmaI and XbaI, and by replacing it with the coding region of a cytomegalovirus (CMV) promoter digested from the pcDNA1 vector (Invitrogen) using NruI and XbaI. Finally, the cDNA of NDUFAF1 was ligated behind the CMV promoter in the KpnI and PstI restriction sites of the modified baculovirus vector. Approximately 25,000 cells were spotted on a 13-mm coverslip, and after 24 h cells were infected with the appropriate virus and cultured for another 48 h. (ref Visch JBC) and (Kost TA, Condreay JP, Jarvis DL. 2005. Baculovirus as versatile vectors for protein expression in insect and mammalian cells. Nat Biotechnol. May;23(5):567-75)

NDUFAF1 expression will be tested by western blotting using a specific antibody against this protein raised in rabbit (see above). Complex I expression will be tested by blue native electrophoresis in combination with an in-gel activity assay and western blotting using specific complex I antibodies (see above).

We are progressing considerably in trying to validate the proof of principle (genetic constructs are ready, baculovirus system is up and running, patient cell lines are available, estimated time of result 3-6 months).

### Example 3

Analysis of the expression pattern of any of the known assembly chaperones of oxidative phosphorylation complexes I, II, III, IV and V by immunodetection on Western blots of 2D BN/SDS-PAGE gels as a diagnostic tool for assembly disturbances:
The expression pattern of any of the known assembly chaperones of oxidative phosphorylation complexes I, II, III, IV and V will be analysed on Western blots of two-dimensional blue-native/SDS-PAGE gels by immunodetection to provide a diagnostic tool for the screening for assembly disturbances of oxidative phosphorylation complexes and related diseases. An example of this method is given below for complex I assembly factor NDUFAF1.

### generation of NDUFAF1 antibody.

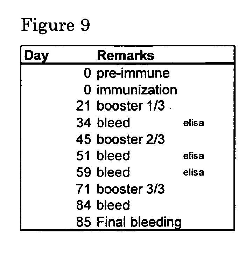

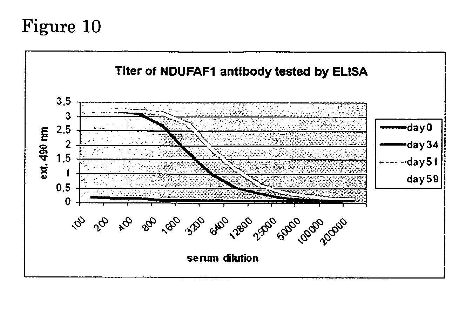

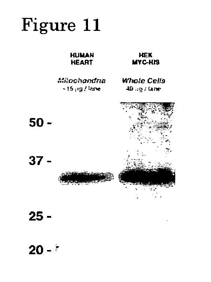

To produce antibodies against NDUFAF1 for immunodetection, two oligopeptides were selected. The first peptide corresponds to the mid-portion of the NDUFAF1 sequence, amino acids 43V-57G; the second peptide corresponds to the C-terminus of the protein, amino acids 314F-327K. A mixture of both peptides was coupled to keyhole limpet hemocyanin, serving as an immunogenic carrier. Rabbits were immunised with an injection of the antigen-carrier conjugate, followed by three subsequent boosters, one every three weeks. The two antisera were collected from a final bleeding and tested for specific detection of the NDUFAF1 protein by Western blotting. Both antisera displayed comparable specificity. See figures 9-11.

### Two-dimensional blue-native/SDS-PAGE electrophoresis of mitochondrial protein

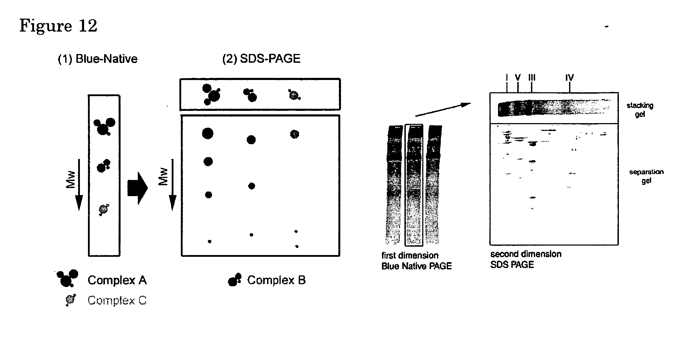

Two-dimensional blue-native SDS-PAGE (2D BN/SDS-PAGE) has proven to be a reliable method for the separation and analysis of native protein complexes. Initial application of this method to visualise complex I assembly intermediates in several complex I deficient cell lines has allowed our lab to verify our assembly model in humans. This method entails the separation of native mitochondrial protein complexes in the first dimension using the dye Serva blue G after which the lane is excised and used for a second dimension. This second dimension SDS denatures the native complexes to separate all peptide constituents of the native complexes (see figure 12). Using this method, all oxidative phosphorylation complexes can be separated and assembly intermediates can be visualised.

### Immunodetection on blots of 2D BN/SDS-PAGE gels to screen for differences in the expression pattern of an OXHPOS assembly protein

We have demonstrated that NDUFAF1 expression differs greatly between membrane arm and peripheral arm CI subunit patients. Prior analysis of the NDUFAF1 expression pattern using this method for CI deficient patients will narrow down the amount of subunits that need to be sequenced for mutations.

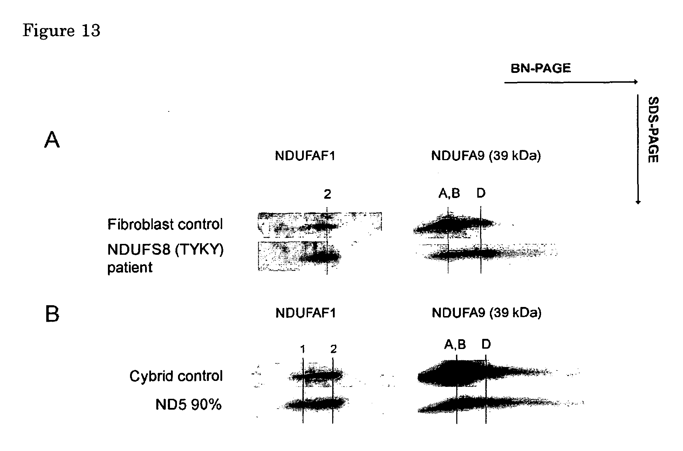

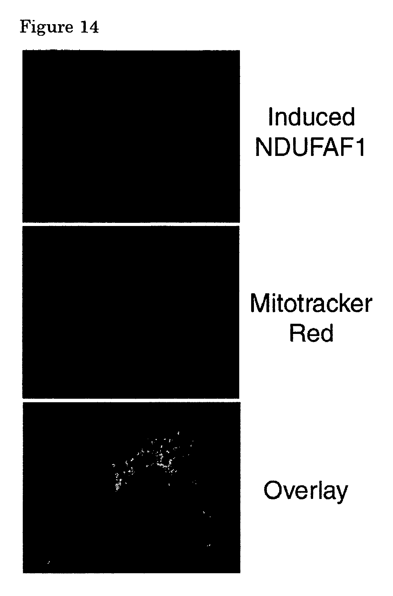

We have analysed the expression pattern of NDUFAF1 using the blue-native electrophoresis method in combination with immunodetection using the NDUFAF1 antibody. Comparison between two classes of patients, one representing cell lines containing mutations in a subunit of the peripheral arm of complex I, the other representing cell lines containing mutations in a subunit of the membrane arm of complex I (figure 13).

By analogy, this analysis can be extrapolated to any of the known assembly chaperones of oxidative phosphorylation complexes I, II, III, IV and V. Diagnostic expression analysis of assembly chaperones of oxidative phosphorylation complexes I, II, III, IV and V will be analysed by a) determination of the amount of chaperone by immunoblotting b) determination of the by 2D BN/SDS-PAGE expression profile by immunoblotting.

By pre-classification of the assembly disturbance using this method, the amount of components of oxidative phosphorylation complexes that need to be sequenced for mutation will be drastically reduced and diagnosis of the disorder can be accelerated.

### Protocols

### ELISA

ELISA for measuring antibody affinity was performed according to standarised protocols [Rennard SI, Berg R, Martin GR, Foidart JM, Robey PG (1980) Enzyme-linked immunoassay (ELISA) for connective tissue components. Anal Biochem 104:205-214].

### Digitonin isolation and solubilisation of mitochondria

Approximately 1 x 106 trypsin-harvested cells were pelleted and resuspended in 100 µl of cold PBS to which 100 µl of 4% digitonin (w/v) was added to achieve a final concentration of 2% digitonin. This sample was shortly vortexed and incubated on ice for 10 minutes to solubilize cell membranes. After this, 1 ml of cold PBS was added and the sample was centrifuged at 10000 g for 10 minutes at 4°C to obtain a mitochondria-enriched organelle pellet. To remove traces of digitonin, this pellet was washed twice with 1 ml of cold PBS. Mitochondrial proteins were solubilised by the addition of 100 µl of AC/BT (1.5 M aminocaproic acid, 75 mM Bis-Tris pH 7.0) and 20 µl of 10% n-dodecyl β-D-maltoside (w/v) and incubation on ice for 10 minutes. The solubilised proteins are retained in the supernatant after centrifugation at 10000 g for 25 minutes at 4°C, which was used for further analysis.

### Gel electrophoresis and in-gel activity assays

The protein concentration for BN-PAGE and SDS-PAGE was determined in the n-dodecyl β-D-maltoside solubilised supernatants before adding coomassie blue containing sample buffer, using a MicroBCA protein assay kit (Pierce). Blue-native 5-15% gradient gels were loaded with 40 µg of digitonin-isolated mitochondria and, after electrophoresis, were further processed for Western blotting, second dimension 10% SDS-PAGE or in-gel activity assays as described earlier [Nijtmans LGJ, Henderson NS & Holt IJ (2002) Blue Native electrophoresis to study mitochondrial and other protein complexes. Methods 26, 327-334]. SDS-PAGE analysis was performed by loading 40 µg of protein/lane on 10% SDS-PAGE gels as described before [Ugalde C, Vogel R, Huijbens R, van den Heuvel B, Smeitink J & Nijtmans L (2004) Human mitochondrial complex I assembles through the combination of evolutionary conserved modules: a framework to interpret complex I deficiencies. Hum Mol Genet 13, 2461-2472].

### Blotting, detection

Blue-native and SDS gels were blotted onto PROTRAN Nitrocellulose Transfer Membrane (Schleicher and Schuell BioScience) according to standard procedures. Primary antibodies used are against NDUFA9 (39 kDa) (Molecular Probes) and NDUFAF1. Secondary antibodies used are Goat Anti-Rabbit PerOxidase (GARPO) and Goat Anti-Mouse PerOxidase (GAMPO) (Molecular Probes). Signal detection was performed using the ECL® plus Western Blotting Detection System (Amersham Biosciences). After ECL, the blots were exposed to X-OMAT UV film (KODAK). ECL signals were quantified using the ImagePro-Plus 4.1 image analysis software (Media Cybernetics, Silver Spring, MD, USA).

### Example 4

### Detection of substances that can increase the intracellular amount of any of the known assembly chaperones of oxidative phosphorylation complexes I, II, III, IV and V

Control fibroblasts and in five patients derived fibroblast cell lines with a decreased amount of fully assembled complex I, which are available in our centre, are screened. (Janssen R et al. Genetic defects in the oxidative phosphorylation (OXPHOS) system.Expert Rev Mol Diagn. 2004 Mar;4(2):143-56. Review gives an overview of available cell lines). Also myoblasts cells with a mitochondrial disorder and model cells in which mitochondrial function has been impaired by siRNA treatment are used for testing compounds. Example compounds are food derivatives, vitamins and vitamin derivatives, radical scavengers (Grad and Lemire, Mitochondrial complex I mutations in Caenorhabditis elegans produce cytochrome c oxidase deficiency, oxidative stress and vitamin-responsive lactic acidosis. Hum Mol Genet. 2004 Feb 1;13(3):303-14.)

Immunodectection Assays: ELISA, electrophoresis, and immunohistochemistry are used to screen for components which will increase expression of assembly chaperones of oxidative phosphorylation complexes I, II, III, IV and V

### siRNA treatment to create model system for mitochondrial deficiency siRNA transfection

For transfection, HeLa cells were plated in DMEM supplemented with 10% FCS (without antibiotics) in 24-well plates with a cell density of 1.5 x 104 cells per well. The next day, cells were transfected with siRNA duplex (control: Cyclophilin B (Dharmacon), NDUFAF1: #1 antisense strand: 5'-ACUAACAUCAGGCUUCUCC dTdT -3', #2 antisense strand: 5'-UAACUAUACAUCUGAUUCG dTdT -3') in 3 µl oligofectamine (Invitrogen) to achieve a final concentration of 10 nM siRNA per well. Cells were incubated at 37°C in a CO2 incubator for 48-72 hours until they were ready to assay for gene knockdown or to perform a second transfection.

### Immunofluorescence assay

A culture of a TO/NDUFAF1 containing HEK293 T-RExtm clone was seeded onto a coverslip in a 24-wells plate and grown overnight to achieve a confluency of 50% on the day of the assay. Cells were prestained with Mitotracker Red (Molecular Probes) prior to paraformaldehyde fixation. For fluorescence imaging, cell preparates were attached to the stage of an inverted microscope (Axiovert 200 M, Carl Zeiss, Jena, Germany) equipped with a x63 Plan NeoFluar (NA 1.25) objective (Carl Zeiss). Alexa Fluor® 488 and Mitotracker Red were excited at 488 nm and 570 nm respectively, using a monochromator (Polychrome IV, TILL Photonics, Gräfelfing, Germany). Fluorescence emission light was either directed by a 505DRLPXR dichroic mirror (Omega Optical Inc., Brattleboro, USA) through a 535AF26 emission filter (Alexa Fluor® 488) or by a 595DRLP dichroic mirror through a 645AF75 emission filter (Mitotracker Red) onto a CoolSNAP HQ monochrome CCD-camera (Roper Scientific, Vianen, The Netherlands). All hardware was controlled with Metafluor 6.0 software (Universal Imaging Corporation, Downingtown, USA) running on a PC equipped with 1 Gb RAM running Windows XP Professional.

### ELISA

ELISA for measuring antibody affinity was performed according to standarised protocols [ Rennard SI, Berg R, Martin GR, Foidart JM, Robey PG (1980) Enzyme-linked immunoassay (ELISA) for connective tissue components. Anal Biochem 104:205-214].

### Blotting, detection

Blue-native and SDS gels were blotted onto PROTRAN Nitrocellulose Transfer Membrane (Schleicher and Schuell BioScience) according to standard procedures. Primary antibodies used are against NDUFA9 (39 kDa) (Molecular Probes) and NDUFAF1. Secondary antibodies used are Goat Anti-Rabbit PerOxidase (GARPO) and Goat Anti-Mouse PerOxidase (GAMPO) (Molecular Probes). Signal detection was performed using the ECL® plus Western Blotting Detection System (Amersham Biosciences). After ECL, the blots were exposed to X-OMAT UV film (KODAK). ECL signals were quantified using the ImagePro-Plus 4.1 image analysis software (Media Cybernetics, Silver Spring, MD, USA).

### Example 5

Increasing the intracellular amount of any of the known assembly chaperones of oxidative phosphorylation complexes I, II, III, IV and V will lead to an increase in the amount of the targeted complex. Doing so in patient cell lines with an assembly disturbance in any of these complexes can alleviate the assembly disturbance.

We claim the use of any of the known assembly chaperones of oxidative phosphorylation complexes I, II, III, IV and V and/or any substance that can increase their intracellular amount with the purpose of treating mitochondrial disorders, Parkinsons disease and/or related diseases. An example is given below for complex I assembly protein NDUFAF1.

### Baculovirus transfection of NDUFAF1 with the purpose of restoring complex I levels in complex I deficient cell lines

Analogous to the results obtained with the inducible expression system for NDUFAF1, expression of the complex I specific chaperone NDUFAF1 will be increased in control fibroblasts and in five patients derived fibroblast cell lines with a decreased amount of fully assembled complex I, which are available in our centre.Example cell lines that are used are NDUFV1 (R59X/T423M; Schuelke M, Smeitink J, Mariman E, Loeffen J, Plecko B, Trijbels JMF, Stockler-Ipsiroglu S, and van den Heuvel LP. Mutant NDUFV1 subunit of mitochondrial complex I causes leukodystrophy and myoclonic epilepsy. Nat Genet 21: 260-261, 1999), NDUFS1 (R557X/D618N; manuscript in preparation), NDUFS2 (R228Q, P229Q, S413P; Loeffen JL, Elpeleg O, Smeitink J, Smeets R, Stockler-Ipsiroglu S, Mandel H, Sengers R, Trijbels JMF, and van den Heuvel LP. Mutations in the complex I NDUFS2 gene of patients with cardiomyopathy and encephalomyopathy. Ann Neurol 49: 195-201, 2001), NDUFS4 (K158fs, VPEEHI67/VEKSIstop, R106X: two unrelated patients; de SM, van den Heuvel LP, Janssen AJ, Smeets RJ, Buskens CA, DeMeirleir L, Van Coster R, Baethmann M, Voit T, Trijbels JMF, and Smeitink JAM. Combined enzymatic complex I and III deficiency associated with mutations in the nuclear encoded NDUFS4 gene. Biochem Biophys Res Commun 275: 63-68, 2000; Loeffen JL, Elpeleg O, Smeitink J, Smeets R, Stockler-Ipsiroglu S, Mandel H, Sengers R, Trijbels JMF, and van den Heuvel LP. Mutations in the complex I NDUFS2 gene of patients with cardiomyopathy and encephalomyopathy. Ann Neurol 49: 195-201, 2001; Van den Heuvel LP, Ruitenbeek W, Smeets R, Gelman-Kohan Z, Elpeleg O, Loeffen J, Trijbels JMF, Mariman E, de Bruijn D, and Smeitink JAM. Demonstration of a new pathogenic mutation in human complex I deficiency: a 5-bp duplication in the nuclear gene encoding the 18-kD (AQDQ) subunit. Am J Hum Genet 62: 262-268, 1998; manuscript in preparation), NDUFS7 (V122M; Triepels RH, van den Heuvel LP, Loeffen JL, Buskens CA, Smeets RJ, Rubio Gozalbo ME, Budde SM, Mariman EC, Wijburg FA, Barth PG, Trijbels JMF, and Smeitink JAM. Leigh syndrome associated with a mutation in the NDUFS7 (PSST) nuclear encoded subunit of complex I. Ann Neurol 45: 787-790, 1999), NDUFS8 (P79L/R102H, R94C; Loeffen JL, Smeitink JAM, Triepels R, Smeets R, Schuelke M, Sengers R, Trijbels JMF, Hamel B, Mullaart R, and van den Heuvel LP. The first nuclear-encoded complex I mutation in a patient with Leigh syndrome. Am J Hum Genet 63: 1598-1608, 1998 , manuscript in preparation) Because primary human skin fibroblasts are refractory to most conventional transfection protocols, we use a baculoviral system to over-express NDUFAF1 in these cells.

After transfection, immunodetection of the intracellular amount of NDUFAF1 and complex I will be performed to assay for over-expression and compensation for the CI deficiency as described below. For protocols regarding the generation of the required NDUFAF1 antibody we refer to other parts of the description..

### Protocols

### Baculovirus transfection of NDUFAF1

The protocol described is derived from the baculovirus transfection system used in [Visch HJ, Rutter GA, Koopman WJ, Koenderink JB, Verkaart S, de Groot T, Varadi A, Mitchell KJ, van den Heuvel LP, Smeitink JA, Willems PH (2004) Inhibition of mitochondrial Na+-Ca2+ exchange restores agonist-induced ATP production and Ca2+ handling in human complex I deficiency. J Biol Chem 279:40328-4036].

The baculovirus system, which is normally used for protein production in Spodoptera frugiperda 9 insect cells, is made suitable for protein expression in mammalian cells by first removing the herpes simplex virus thymidine kinase polyadenylation signal from the pFastBacTMDual vector (Invitrogen) using AccI and XhoI, by next, after blunting and ligation, removing both the p10 and polyhedron promoter with SmaI and XbaI, and by replacing it with the coding region of a cytomegalovirus (CMV) promoter digested from the pcDNA1 vector (Invitrogen) using NruI and XbaI. Finally, the cDNA of NDUFAF1 is ligated behind the CMV promoter in the KpnI and PstI restriction sites of the modified baculovirus vector. Control and patient cell lines are grown to 50% confluency and after 24 h cells are infected with the appropriate virus and cultured for another 48 h. These cells are harvested for isolation of mitochondria and analysis of protein content; e.g. over-expression of NDUFAF1 and increase of CI amount and activity.

### Gel electrophoresis and in-gel activity assays

The protein concentration for BN-PAGE and SDS-PAGE is determined in the n-dodecyl β-D-maltoside solubilised supernatants before adding coomassie blue containing sample buffer, using a MicroBCA protein assay kit (Pierce). Blue-native 5-15% gradient gels are loaded with 40 µg of digitonin-isolated mitochondria and, after electrophoresis, are further processed for Western blotting, second dimension 10% SDS-PAGE or in-gel activity assays as described earlier [Nijtmans LGJ, Henderson NS & Holt IJ (2002) Blue Native electrophoresis to study mitochondrial and other protein complexes. Methods 26, 327-334]. SDS-PAGE analysis is performed by loading 40 µg of protein/lane on 10% SDS-PAGE gels as described before [Ugalde C, Vogel R, Huijbens R, van den Heuvel B, Smeitink J & Nijtmans L (2004) Human mitochondrial complex I assembles through the combination of evolutionary conserved modules: a framework to interpret complex I deficiencies. Hum Mol Genet 13, 2461-2472].

### Blotting, detection

Blue-native and SDS gels are blotted onto PROTRAN Nitrocellulose Transfer Membrane (Schleicher and Schuell BioScience) according to standard procedures. Primary antibodies used are against NDUFA9 (39 kDa) (Molecular Probes) and NDUFAF1. Secondary antibodies used are Goat Anti-Rabbit PerOxidase (GARPO) and Goat Anti-Mouse PerOxidase (GAMPO) (Molecular Probes). Signal detection is performed using the ECL® plus Western Blotting Detection System (Amersham Biosciences). After ECL, the blots are exposed to X-OMAT UV film (KODAK). ECL signals are quantified using the ImagePro-Plus 4.1 image analysis software (Media Cybernetics, Silver Spring, MD, USA)

### Brief description of the drawings

Figure 1.
   Western blot analysis of cellular fractionation of HEK293 cells. A 10% SDS-PAGE gel was loaded with 40 µg protein/lane as follows. Lane 1, total cell protein extract. Lane 2, cytoplasmic protein extract. Lane 3, mitochondrial protein extract. The Western blot of this gel was treated with antibodies against NDUFAF1, GAPDH (cytoplasmic marker), COXII and porin (mitochondrial markers) and HSP70 (loading control).
Figure 2.
   Western blot analysis of induction of NDUFAF1 in HEK293 cells. A 10% SDS-PAGE gel was loaded with 40 µg protein/lane of a mitochondrial protein extract of an inducible HEK293 clone and blotted. Cells were induced with 0.1 µg/ml of doxycycline for 0 (control), 1, 2, 4, 24, 48 and 72 hours. Antibodies used are against NDUFAF1 and complex I membrane arm subunit ND1. Induced and endogenous NDUFAF1 and ND1 signals are indicated on the right. Quantification of the ECL signals is shown in the histogram below, each bar corresponding to the lane above. The signal is expressed as percentage of the 0 hour time point signal for both endogenous NDUFAF1 (white bars) and induced NDUFAF1 (black bars).
Figure 3.
   Immunofluorescence mitochondrial localisation of induced NDUFAF1. Cells that are induced for NDUFAF1 expression by 1 µg/ml of doxycycline for 24 hours were treated with either anti-myc antibody coupled to Alexa Fluor 488 to verify presence of induced NDUFAF1 (top panel) or mitochondrial control Mitotracker Red (middle panel). The bottom panel shows an overlay of the two signals.
Figure 4.
   RNA interference of NDUFAF1 in HeLa cells. A, B and E, HeLa cells were transiently transfected twice for 48 and 48 hours, 48 and 72 hours, and 48 and 96 hours, respectively. Quantification of the signals is represented in the histograms below each panel. Each bar corresponds to the lane above it, representing the percentage of signal compared to the "untreated" signal, corrected for the loading control signal (CoII). A, Western blot of SDS-PAGE gel. B, Western blot of blue-native gel. C, In-gel activity assay. For these panels: Lane 1, untreated cells. Lane 2, mock transfection (no siRNA). Lane 3, transfection with NDUFAF1 siRNA #2 for 48 and 48 hours, consecutively. Lane 4, transfection with NDUFAF1 siRNA #2 for 48 and 72 hours, consecutively. Lane 5, transfection with NDUFAF1 siRNA #2 for 48 and 96 hours, consecutively. Antibodies used are against NDUFAF1, NDUFA9 (complex I) and CoII-70 kDa (complex II, loading control). In-gel activity results are indicated with IGA, Western blot results are indicated with WB.
Figure 5.
   2D BN/SDS-PAGE expression analysis of a mitochondrial protein extract from HEK293 cells. 40 µg protein per lane was loaded on a 5-15% blue-native gel (1st dimension) followed by 2nd dimension separation using 10% SDS-PAGE gels and Western blotting. Antibodies used are against NDUFAF1 and NDUFA9 (39 kDa), indicated at the left of each panel. CI refers to fully assembled complex I (complex A in Ugalde et al., [14]). Numbers 1 and 2 refer to NDUFAF1 complexes of 700 and 600 kDa, respectively.
Figure 6.
   2D BN/SDS-PAGE expression analysis of mitochondrial protein extracts from HEK293 control cells and a NDUFAF1 inducible HEK293 clone. The inducible clone was induced for NDUFAF1 expression for 1, 2 and 4 hours with 1 µg /ml of doxycycline. 40 µg protein per lane was loaded on a 5-15% blue-native gel (1st dimension) followed by 2nd dimension separation using 10% SDS-PAGE gels and Western blotting. Anti NDUFAF1 antibody was used for immunoblot detection. NDUFAF1 containing intermediates are indicated with 1 (700 kDa) and 2 (600 kDa). Induced and endogenously expressed NDUFAF1 are indicated on the right with "induced" and "endogenous", respectively to differentiate between the endogenous protein and the slightly larger tagged induced protein.
Figure 7.
   NDUFAF1 expression in two complex I deficient patients (panels A and B) and in a conditional complex I assembly system (panel C). Mitochondrial pellets were solubilised and 40 µg of protein was loaded onto a 5-15% BN gel. Expression profiles were analysed by 2D BN/SDS-PAGE, Western blotting and antibody incubation. Antibodies used are against NDUFAF1 and NDUFA9 (39 kDa). Assembly stages described in ref. 14 are indicated at the top by A, B and D. NDUFAF1 containing complexes are indicated by 1 (700 kDa) and 2 (600 kDa). Panel A: expression analysis of control 143B osteosarcoma cells and a complex I peripheral arm subunit patient cell line (NDUFS8 mutation, manuscript in preparation)). Panel B: expression analysis of control cybrids (cybrid control) and a complex I membrane arm subunit patient cell line (ND5 mutation D393G (see reference 19)) with heteroplasmy level of 90%. Panel C: expression analysis of mitochondrial protein extracts from 143B osteosarcoma cells inhibited for mitochondrial protein synthesis by treatment with 15 µg/ml of doxycycline for 5 days. Samples were taken at 0, 3, 6, 12, 24, 48 hours and 5 days after release of doxycycline inhibition. Control (143B osteosarcoma cells) panel is at the top.
Figure 8.
   Overexpression of NDUFAF1 leads to a specific increase in the amount and activity of complex I. Four time points after washing away a pulse of 4 hours of induction of expression with 1 ug/ml of doxycycline are shown (0, 24, 48 and 72 hours of "chasing"). Complex I in-gel activity is indicated by "CoI IGA". Complex I is indicated by "CoI 39 kDa". Complex II is indicated by "CoII 70 kDa". Complex III is indicated by "CoIII CoreII". Complex IV is indicated by "CoIV COXII". Endogenously expressed NDUFAF1 is indicated by "NDUFAF1 end". Induced NDUFAF1 is indicated by "NDUFAF1 ind".
Figure 9.
   Timecourse of immunization. The rabbit was immunized with both peptides at t=0 and with three subsequent boosters at t=21, t=45 and t=71 days. Blood was obtained from the rabbit at timepoints indicated.
Figure 10.
   Titer of NDUFAF1 antibody measured by ELISA after three subsequent bleedings (34, 51 and 59 days post immunisation).
Figure 11.
   Western blot immunodetection of NDUFAF1 (35 kDa) using the NDUFAF1 antibody. The antibody was tested on Western blots of 10% SDS-PAGE gels loaded with 15 µg of a lysate of human heart mitochondria and with 40 µg of a lysate of whole HEK293 cells. NDUFAF1 is clearly and specifically detected at 35 kDa.
Figure 12. Principle of blue-native/SDS-PAGE. First dimension blue-native separates native protein complexes, which are denatured in the second dimension SDS-PAGE.
Figure 13.
   NDUFAF1 expression on blots of 2D BN/SDS-PAGE gels of a cell line with a mutation in complex I subunit NDUFS8 (A) and with a mutation in complex I subunit ND5 (B). Complex I is indicated on the right panels and detected using an antibody against complex I subunit NDUFA9. NDUFAF1 complex I is more prominent for the ND5 mutation whereas NDUFAF1 complex 2 is more prominent for the NDUFS8 mutation.
Figure 14.
   Detection of substances that increases the amount of complex I in a mitochondrion.
Figure 15.
   Evolutionary conservation of certain amino acids of NDUFAF1.

### References

1. Smeitink J, van den Heuvel L & DiMauro S (2001) The genetics and pathology of oxidative phosphorylation. Nat Rev Gene 2, 342-352.
2. Barrientos A, Barros MH, Valnot I, Rotig A, Rustin P & Tzagoloff A (2002) Cytochrome oxidase in health and disease. Gene 286, 53-63.
3. Janssen R, Smeitink J, Smeets R & van den Heuvel L (2002) CIA30 complex I assembly factor: a candidate for human complex I deficiency? Hum Genet 110, 264-270.
4. Grigorieff N (1999) Structure of the respiratory NADH:ubiquinone oxidoreductase (complex I). Curr Opin Struct Biol 9, 476-483.
5. Carroll J, Fearnley IM, Shannon RJ, Hirst J & Walker JE (2003) Analysis of the subunit composition of complex I from bovine heart mitochondria. Mol Cell Proteomics 2, 117-26.
6. Hirst J, Carroll J, Fearnley IM, Shannon RJ & Walker JE (2003) The nuclear encoded subunits of complex I from bovine heart mitochondria. Biochim Biophys Acta 1604, 135-150.
7. Vinogradov AD (2001) Respiratory complex I: structure, redox components, and possible mechanisms of energy transduction. Biochemistry 66, 1086-97.
8. Cardol P, Matagne RF & Remacle C (2002) Impact of mutations affecting ND mitochondria-encoded subunits on the activity and assembly of complex I in Chlamydomonas. Implication for the structural organization of the enzyme. J Mol Biol 319, 1211-1221.
9. Stroh A, Anderka O, Pfeiffer K, Yagi T, Finel M, Ludwig B & Schagger H (2004) Assembly of respiratory complexes I, III, and IV into NADH oxidase supercomplex stabilizes complex I in Paracoccus denitrificans. J Biol Chem 279, 5000-5007.
10. Yadava N, Houchens T, Potluri P & Scheffler IE (2004) Development and characterization of a conditional mitochondrial complex I assembly system. J Biol Chem 279, 12406-12413.
11. Videira A (1998) Complex I from the fungus Neurospora crassa. Biochim Biophys Acta 1364, 89-100.
12. Schulte U (2001) Biogenesis of respiratory complex I. J Bioenerg Biomembr 33, 205-212.
13. Antonicka H, Ogilvie I, Taivassalo T, Anitori RP, Haller RG, Vissing J, Kennaway NG & Shoubridge EA (2003) Identification and characterization of a common set of complex I assembly intermediates in mitochondria from patients with complex I deficiency. J Biol Chem 278, 43081-43088.
14. Ugalde C, Vogel R, Huijbens R, van den Heuvel B, Smeitink J & Nijtmans L (2004) Human mitochondrial complex I assembles through the combination of evolutionary conserved modules: a framework to interpret complex I deficiencies. Hum Mol Genet 13, 2461-2472.
15. Kuffner R, Rohr A, Schmiede A, Krull C & Schulte U (1998) Involvement of two novel chaperones in the assembly of mitochondrial NADH:Ubiquinone oxidoreductase (complex I). J Mol Biol 283, 409-417.
16. Duarte M & Videira A (2000) Respiratory chain complex I is essential for sexual development in neurospora and binding of iron sulfur clusters are required for enzyme assembly. Genetics 156, 607-615.
17. Vahsen N, Cande C, Briere JJ, Benit P, Joza N, Larochette N, Mastroberardino PG, Pequignot MO, Casares N, Lazar V, Feraud O, Debili N, Wissing S, Engelhardt S, Madeo F, Piacentini M, Penninger JM, Schagger H, Rustin P & Kroemer G (2004) AIF deficiency compromises oxidative phosphorylation. EMBO J 23, 4679-4689.
18. Nijtmans LGJ, Henderson NS & Holt IJ (2002) Blue Native electrophoresis to study mitochondrial and other protein complexes. Methods 26, 327-334.
19. Corona P, Antozzi C, Carrara F, D'Incerti L, Lamantea E, Tiranti V & Zeviani M (2001) A novel mtDNA mutation in the ND5 subunit of complex I in two MELAS patients. Ann Neurol 49, 106-110.

### Table 1

### Part I: mitochondrial disorders

Review on specific mitochondrial disorders:
DiMauro S, Hirano M. 2005. Mitochondrial encephalomyopathies: an update. Neuromuscular Disorders 15: 276-286 (and references cited herein).

For a clinical overview of known mitochondrial disorders (up to date until 13/9/5)
(http://www.neuro.wustl.edu/neuromuscular/mitosvn.html#clinicalsyndromes), presented here:

| **MITOCHONDRIAL DISORDERS: CLINICAL SYNDROMES** | |
|---|---|
| Adult onset | Myopathy syndromes |
| Alexander disease: NDUFV1; 11q13 | Infantile myopathies |
| Alzheimer/Parkinsonism | Fatal: mtDNA depletion |
| Amino Acid disorders: Nuclear mutations | "Later-onset": mtDNA depletion Inflammatory myopathy |
| Ataxias Barth: Tafazzins; Xp28 | Inclusion body myositis: Mpl mtDNA deletions |
| Cardiomyopathy | mtDNA depletion: "Later-onset" |
| Carnitine disorders | PM + COX- muscle fibers: Mpl |
| Cartilage-Hair hypoplasia | mtDNA deletions |
| CNS | NARP/MILS: mt ATPase6 |
| Infantile & Childhood onset | Neoplasms |
| Syndromes | Neuropathy syndromes |
| Congenital muscular dystrophy: | CMT 2A (MFN2) |
| Nuclear mutation | CMT 4A |
| Cramps | Sensory neuropathy: Recessive; |
| Deafness | Sporadic |
| Maternal (mtDNA): Point mutations | Occipital horn syndrome: ATPase |
| Syndromic (HAM; MELAS; | 7a; Xq12 |
| MERRF): tRNA | Ophthalmoplegia, External (PEO) |
| Non-syndromic & amino-glycoside induced: 12s rRNA | Dominant: Multiple mtDNA deletions |
| Nuclear mutations | Maternal: mtDNA point mutations |
| DIDMOAD: WFS1; 4p16 | Recessive: mtDNA depletion; |
| Deafness-Dystonia: DDP protein; | Multiple mtDNA deletions |
| Xq22 | Sporadic: Single mtDNA deletion |
| Diabetes | ? Immune (HyperThyroid) |
| Dystonia | Optic atrophy |
| Encephalopathies | Paraganglioma |
| Fatigue & Exercise intolerance | PGL1: SDH Subunit D; 11q23 |
| Friedreich ataxia: Frataxin; 9q13 | PGL3: SDH Subunit C; 1q21 |
| Functional defects | PGL + Pheochromocytoma: SDH |
| Gastrointestinal | Subunit B; 1p36 |
| HAM: mtDNA tRNA Ser | Parkinson's |
| Huntington's chorea | Pearson's: mtDNA deletion |
| Infantile CNS: mtDNA & Nuclear mutations | Rhabdomyolysis: mtDNA Selenium deficiency |
| Kearns-Sayre: Single mtDNA deletion | Spastic paraparesis SPG7: Paraplegin; 16q24 |
| Leber's optic neuropathy: mtDNA | SPG13: HSPD1; 2q24 |
| NADH-Dehydrogenase + | HHH: Ornithine transporter; |
| Leigh's syndrome: mtDNA & Nuclear mutations | 13q14 Spinal muscular atrophy: TK2; |
| Leukodystrophy | 16q22 |
| Longevity | Stuve-Wiedemann syndrome: 1p34 |
| Maple syrup urine disease | Sudden infant death (SIDS): |
| MELAS: mtDNA tRNA Leu + other | mtDNA tRNA Leu |
| Menkes: ATPase 7a; Xq12 | Systemic disorders |
| MERRF: mtDNA tRNA Lys & Ser | Toxic |
| MNGIE: Thymidine phosphorylase; | AZT (Zidovudine) |
| 22q13 | Copper |
| Multiple symmetric lipomatosis: | Germanium |
| mtDNA tRNA Lys & Nuclear | Valproate: Precipitates seizures in |
| Myalgias | MELAS |
| Myoglobinuria | Wilson's disease: ATPase 7B; 13q14 |
| | Wolfram |
| | WFS1; 4p16 |
| | WFS2; 4q22 |

### Part II: OXPHOS disorders

For an up-to-date review on OXPHOS genetic defects:
Janssen RJ, van den Heuvel LP, Smeitink JA. 2004. Genetic defects in the oxidative phosphorylation (OXPHOS) system. Expert Rev Mol Diagn 4: 143-156.

Tables presented in this review:

### Mitochondrial-encoded genes involved in oxidative phosphorylation (OXPHOS) deficiency:

### Gene (mutation), affected biochemical mechanism and clinical presentation

### Subunits of the OXPHOS system

ND1 (G3460A) OXPHOS complex I LHON
ND1 (A3796G) OXPHOS complex I Adult-onset dystonia, spasticity and core-type myopathy
ND2 (G5244A) OXPHOS complex I LHON
ND3 (T10191C) OXPHOS complex I Progressive epilepsy, stroke-like episodes, optic atrophy and cognitive decline
ND4 (G11778A) OXPHOS complex I LHON
ND4 (G11832A) OXPHOS complex I Exercise intolerance
ND4L (T10663C) OXPHOS complex I LHON
ND5 (T12706C) OXPHOS complex I Leigh syndrome
ND5 (A13084T) OXPHOS complex I Leigh-MELAS overlap syndrome
ND5 (G13513A) OXPHOS complex I Leigh-like syndrome
ND5 (A13514G) OXPHOS complex I MELAS-like
ND5 (G13708A) OXPHOS complex I LHON
ND6 (G14453A) OXPHOS complex I MELAS
ND6 (G14459A) OXPHOS complex I LHON and dystonia
ND6 (G14459A) OXPHOS complex I Leigh syndrome
ND6 (C14482G, C14482A,T14484C, A14495G) OXPHOS complex I LHON CYTB (de114787-14790) OXPHOS complex III Parkinsonism/MELAS overlap syndrome
CYTB (G15059A) OXPHOS complex III Progressive exercise muscle intolerance, proximal limb weakness and myoglobinuria
CYTB (G15242A) OXPHOS complex III Exercise intolerance, lactic acidosis and mitochondrial encephalomyopathy
CYTB (G15257A) OXPHOS complex III LHON
CYTB (C15452A) OXPHOS complex III Ischemic cardiopathy
CYTB (G15615A) OXPHOS complex III Progressive exercise muscle intolerance
COXI (5-bp del 5'UTR) OXPHOS complex IV Isolated motor neurone disease
COXI (T6721C, T6742C) OXPHOS complex IV Sideroblastic anemia
COXI (G6930A) OXPHOS complex IV Multisystem mitochondrial disorder COXI (G7444A) OXPHOS complex IV LHON
COXII (T7671A) OXPHOS complex IV Proximal myopathy and lactic acidosis COXII (G7896A) OXPHOS complex IV Early-onset multisystem disorder COXIII (15-bp del) OXPHOS complex IV Recurrent myoglobinuria
COXIII (ins9537C) OXPHOS complex IV Leigh-like syndrome
COXIII (G9804A, G9438A) OXPHOS complex IV LHON
COXIII (T9957C) OXPHOS complex IV MELAS
ATP6 (T8993G) OXPHOS complex V NARP
ATP6 (T8993G) OXPHOS complex V MILS
ATP6 (T9101C) OXPHOS complex V LHON

### Transfer RNA

tRNA Leu(UUR) (A3243G,T3271C, T3291C) Mitochondrial translation MELAS
tRNA Leu(UUR) (A3243G) Mitochondrial translation MERRF/MELAS overlap syndrome
tRNA Leu(UUR) (A3243G) Mitochondrial translation Diabetes and deafness
tRNA Leu(UUR) (A3243G) Mitochondrial translation CPEO; Kearns-Sayre syndrome
tRNA Leu(UUR) (C3303T) Mitochondrial translation Cardiomyopathy and/or mitochondrial myopathy
tRNA Leu(UUR) (T3250C, A3302G) Mitochondrial translation Mitochondrial myopathy tRNA Leu(CUN) (A12320G) Mitochondrial translation Mitochondrial myopathy
tRNA Leu(CUN) (T12297C) Mitochondrial translation Dilated cardiomyopathy tRNA Leu(CUN) (G12315A) Mitochondrial translation CPEO
tRNA Leu(CUN) (G12301A) Mitochondrial translation Sideroblastic anemia
tRNA Lys (A8344G, A8296G) Mitochondrial translation MERRF
tRNA Lys (T8316C) Mitochondrial translation MELAS
tRNA Lys (G8363A) Mitochondrial translation Cardiomyopathy and hearing loss; Leigh syndrome
tRNA Lys (G8342A) Mitochondrial translation CPEO
tRNA Ile (A4300G) Mitochondrial translation Hypertrophic cardiomyopathy
tRNA Ile (A4267G) Mitochondrial translation Mitochondrial myopathy, ataxia and sensorineural deafness
tRNA Ile (T4285C) Mitochondrial translation CPEO
tRNA Ser(UCN) (A7445G) Mitochondrial translation Sensorineural deafness
tRNA Ser(UCN) (T7512C) Mitochondrial translation MERRF/MELAS overlap syndrome
tRNA Ser(AGY) (C12258A) Mitochondrial translation Diabetes and deafness
tRNA Ala (T5628C) Mitochondrial translation CPEO
tRNA Cys (A5814G) Mitochondrial translation Hypertrophic cardiomyopathy; encephalomyopathy
tRNA Asp (A7543G) Mitochondrial translation Myoclonic epilepsy
tRNA Glu (T14687C) Mitochondrial translation Mitochondrial myopathy and lactic acidosis, retinopathy and respiratory failure
tRNA Glu (T14709C) Mitochondrial translation Mitochondrial myopathy and diabetes mellitus
tRNA Phe Mitochondrial translation MELAS
tRNA Phe (T618C) Mitochondrial translation Mitochondrial myopathy tRNA Gly (T9997C) Mitochondrial translation Hypertrophic cardiomyopathy; encephalomyopathy
tRNA Met (T4409C) Mitochondrial translation Mitochondrial myopathy
tRNA Asn (G5703A) Mitochondrial translation CPEO
tRNA Pro (G15990A) Mitochondrial translation Mitochondrial myopathy
tRNA Gln (ins4370A) Mitochondrial translation Myopathy
tRNA Gln (G4332A) Mitochondrial translation MELAS
tRNA Thr (G15915A) Mitochondrial translation Mitochondrial encephalomyopathy
tRNA Val (G1642A) Mitochondrial translation MELAS
tRNA Val (1644) Mitochondrial translation Leigh syndrome
tRNA Trp (ins5537T) Mitochondrial translation Leigh syndrome
tRNA Tyr (G5877A) Mitochondrial translation CPEO

### Ribosomal RNA

12S rRNA (A1555G) Mitochondrial transcription Nonsyndromic sensorineural deafness [139]
12S rRNA (T1095C) Mitochondrial transcription Sensorineural deafness, Parkinsonism and neuropathy [140]
16S rRNA (C3093G) Mitochondrial transcription MELAS, diabetes mellitus, hyperthyroidism and cardiomyopathy

### Nuclear-encoded genes involved in oxidative phosphorylation (OXPHOS) deficiency:

### Gene (mutation), affected biochemical mechanism and clinical presentation

### Subunits of the OXPHOS system

NDUFS1 OXPHOS complex I Leigh syndrome and leukodystrophy
NDUFS2 OXPHOS complex I Hypertrophic cardiomyopathy and encephalomyopathy
NDUFS4 OXPHOS complex I Leigh syndrome
NDUFS4 OXPHOS complex I Leigh-like syndrome
NDUFS7 OXPHOS complex I Leigh syndrome
NDUFS8 OXPHOS complex I Leigh syndrome
NDUFV1 OXPHOS complex I Leukodystrophy and myoclonic epilepsy
NDUFV2 OXPHOS complex I Hypertrophic cardiomyopathy and encephalomyopathy
SDHA OXPHOS complex II Leigh syndrome
SDHA OXPHOS complex II Late-onset optic atrophy, ataxia and myopathy
SDHB OXPHOS complex II Hereditary paraganglioma/pheochromocytoma (PGL4)
SDHC OXPHOS complex II Hereditary paraganglioma (PGL3)
SDHD OXPHOS complex II Hereditary paraganglioma/pheochromocytoma (PGL1)
UQCRB OXPHOS complex III Hypoglycemia and lactic academia

### Assembly factors of the OXPHOS complexes

BCS1L OXPHOS complex III Neonatal proximal tubulopathy, hepatic involvement and encephalopathy
BCS1L OXPHOS complex III GRACILE syndrome
SURF-1 OXPHOS complex IV Leigh syndrome
SURF-1 OXPHOS complex IV Leukodystrophy
SURF-1 OXPHOS complex IV Villous atrophy, hypertrichosis, mild neurological involvement
SCO1 OXPHOS complex IV Neonatal-onset hepatic failure and encephalopathy
SCO2 OXPHOS complex IV Encephalopathy and hypertrophic cardiomyopathy
COX10 OXPHOS complex IV Mitochondrial encephalopathy, hypotonia ataxia and seizures
COX15 OXPHOS complex IV Fatal, infantile hypertrophic cardiomyopathy

### OXPHOS system biogenesis

DDP1 TIMM Deafness dystonia syndrome, Mohr-Tranebjaerg syndrome
FRDA (frataxin) Iron homeostasis Friedreich's ataxia
SPG7 (paraplegin) Mitochondrial protein metabolism Hereditary spastic paraplegia (HSP)
TAZ (tafazzin) Cardiolipin metabolism Barth syndrome
OPA1 Mitochondrial inner membrane maintenance Dominant optic atrophy

### Mitochondrial DNA maintenance

ANT1 ADP/ATP transporter adPEO
POLG1 (polymerase-γ A) mtDNA replication adPEO, arPEO
C100RF2 (twinkle) mtDNA replication adPEO
TK2 (thymidine kinase 2) mtDNA replication mtDNA depletion syndrome - myopathic form
DGUOK (deoxyguanosine kinase) mtDNA replication mtDNA depletion syndrome - hepatocerebral form
TP (thymidine phosphorylase) mtDNA replication MNGIE
DNC (deoxynucleotide carrier) mtDNA replication Amish congenital microcephaly (MCPHA)
THTR-1 or SLC19A2 Thiamine transporter, PDH complex, OXPHOS complex I TRMA, Rogers syndrome

### Part III: related diseases

Association mitochondia with Alzheimer:
Günther C, von Hadeln K, Müller-Thomsen T, Alberici A, Binetti G, Christoph Hock, Roger M. Nitsch, Stoppe G., Reiss J., Gal A. and Finckh U. 2004. Possible association of mitochondrial transcription factor A (TFAM) genotype with sporadic Alzheimer disease. Neurosci Lett 369: 219-223.
Bubber P, Haroutunian V, Fisch G, Blass J.P., Gibson G.E Mitochondrial abnormalities in Alzheimer brain: Mechanistic implications. 2005. Ann Neurol 57: 695-703.

Association mitocondria with Parkinson:
Gu G., Reyes, P. F., Golden, G.T., Woltjer R. L., Hulette C., Montine T.J., Zhang J. 2002. Mitochondrial DNA Deletions/Rearrangements in Parkinson Disease and Related Neurodegenerative Disorders. Journal of Neuropathology & Experimental Neurology 61: 634-639.

Association mitochondria with ageing:
Trifunovic A., Wredenberg A.,Falkenberg M.,Spelbrink J.N., Rovio A.T. et al. 2004 Premature ageing in mice expressing defective mitochondrial DNA polymerase. Nature 429: 417-23.

### Table 2

### 1 Human OXPHOS assembly chaperones

### Complex I:

NDUFAF1 (Janssen R, Smeitink J, Smeets R & van den Heuvel L (2002) CIA30 complex I assembly factor: a candidate for human complex I deficiency? Hum Genet 110, 264-270, CIA84: (gi:51593777)

### Complex III

CPB3 (gi:28395057), BCS1 (gi:46397351),

### Complex IV

COX11, COX15. COX17, SCO1, SCO2, SURF1, PET191 (Barrientos A, Barros MH, Valnot I, Rotig A, Rustin P, Tzagoloff A Cytochrome oxidase in health and disease. Gene 2002.Mar.6.;286.(1.):53.-63. 286:53-63)
LRPPRC (Mootha et al. Identification of a gene causing human cytochrome c oxidase deficiency by integrative genomics.Proc Natl Acad Sci U S A. 2003 Jan 21;100(2):605-10.)
COX19, COX16, PET191 (Tay et al. Studies of COX16, COX19, and PET191 in human cytochrome-c oxidase deficiency. Arch Neurol. 2004 Dec;61(12):1935-7.)

### Complex V :

ATP11 (gi:17511865), ATP12 (gi:73917623)

### Multiple complexes

OXA1 gi:38372882
AFG3L, paraplegin, YME1L (Jacobs et al. The mitochondrial inner membrane AAA metalloprotease family in metazoans.FEBS Lett. 2000 Sep 15;481(2):91-5. Review.)

### 2. Evolutionary conserved genes resembling complex I genes:

Note: these are genes are not established assembly chaperones. Only based on conserved evolution we consider these proteins as candidate assembly chaperones. (We believe that there will be more assembly proteins discovered)
GI:40018641, GI:31341340, GI :8895094 (Gabaldon, et al Genes Tracing the evolution of a large protein complex in the eukaryotes, NADH:ubiquinone oxidoreductase (Complex I). J Mol Biol. 2005 May 13;348(4):857-70.)
Mimitin (Tsuneoka et al, A novel Myc-target gene, mimitin, that is involved in cell proliferation of esophageal squamous cell carcinoma.J Biol Chem. 2005 May 20;280(20):19977-85)

### 3. OXPHOS assembly genes known in other species whereof no homologue in human has been described yet.

Complex II : TCM62 (Dibrov and Lemire The Saccharomyces cerevisiae TCM62 gene encodes a chaperone necessary for the assembly of the mitochondrial succinate dehydrogenase (complex II).J Biol Chem. 1998 Nov 27;273(48):32042-8)

complex III: CBP4 (Sc), CBS2 (Sc)

complex IV: COX18 (Sc), COX14 (Sc), COX20 (Sc), PET197 (Sc)

complex V : ATP10(Sc), FMC1 (Sc)

multiple complexes : MBA1 (Sc)

And other proteins that play a role in assembly of OXPHOS complexes Literature references are in between brackets otherwise the gene bank accession number is given (gi:)

## Claims

1. A method for the treatment of a condition that is associated with an oxidative phosphorylation deficiency in an individual comprising providing cells that are affected by said deficiency with an assembly chaperone for a complex involved in oxidative phosphorylation in a mitochondrion.

2. A method according to claim 1, wherein said condition is a condition depicted in table 1.

3. A method according to claim 1 or claim 2, wherein said condition comprises Parkinson, Alzheimer or a mitochondrial disorder.

4. A method according to claim 2, wherein said mitochondrial disorder comprises mitochondrial myopathy, cardiomyopathy, encephalomyopathy, Myoclonic epilepsy, Leigh and Leigh-like syndrome, Leber hereditary optic Neuropathy (LHON), neuropathy ataxia and retinitis pigmentosa (NARP), mitochondrial encephalopathy and stroke-like episodes (MELAS) and myoclonus epilepsy with ragged-red fibers (MERRF)), related diseases (such as Alzheimer's disease, Parkinson's disease, diabetes and deafness) and the process of ageing.

5. A method according to claim 1, wherein said condition comprises aging.

6. A method according to any one of claims 1-5, wherein said assembly chaperone comprises a chaperone of table 2 or a functional part, derivative and/or orthologue thereof

7. A method according to claim 6 wherein said assembly chaperone comprises NDUFAF1, CIA84, mimitin, CPB3, BCS1, COX10, COX11, COX15, COX17, SCO1, SCO2, SURF1, PET191, LRPPRC, ATP11, ATP12, OXA1 or a functional part, derivative and/or orthologue thereof.

8. A method according to any one of claims 1-7, wherein said complex comprises complex I.

9. A method according to any one of claims 1-8, wherein said assembly chaperone comprises NDUFAF1.

10. A cosmetic composition comprising a nucleic acid encoding an assembly chaperone for a complex involved in oxidative phosphorylation in a mitochondrion.

11. A cosmetic composition comprising an assembly chaperone for a complex involved in oxidative phosphorylation in a mitochondrion.

12. A cosmetic composition according to claim 10 or claim 11, further comprising a vehicle for transporting said nucleic acid and/or said protein into a cell.

13. A cosmetic composition according to claim 12, wherein said vehicle comprises a liposome or a viral capsid.

14. A cosmetic composition according to claim 12, wherein said vehicle comprises a cell penetrating peptide.

15. A cosmetic composition according to claim 14, wherein said cell penetrating peptide comprises penetratin, Tat-fragment (48-60), Transportan and amphilic model peptide.

16. A cosmetic composition according to any one of claims 10-15, wherein said assembly chaperone comprises NDUFAF1.

17. A method for analysing an expression profile in a subset of cells of an individual comprising determining in a sample comprising said subset an expression profile of at least two assembly chaperones for at least one complex involved in oxidative phosphorylation in a mitochondrion.

18. A method according to claim 17, wherein said individual is suffering from or is at risk of suffering from Parkinson, Alzheimer or a mitochondrial disorder.

19. A method according to claim 17 or claim 18, wherein at least two of said at least two assembly chaperones are assembly chaperones for different complexes.

20. A method according to any one of claims 17-19, wherein at least one of said assembly chaperones is an assembly chaperone of table 2 or a functional part, derivative and/or orthologue thereof.

21. A method for testing a compound comprising contacting a cell having a decreased amount of a complex involved in oxidative phosphorylation in a mitochondrion with said compound and determining whether the amount of said complex is increased.

22. A method according to claim 21, wherein said amount of said complex is determined by means of electrophoresis and/or immunohistochemistry.

23. A method according to claim 22, wherein said electrophoresis comprises native gel-electrophoresis.

24. A method according to claim 22 or claim 23, wherein said immunohistochemistry comprises detecting binding of binding body specific for NDUFAF1 peptide 313F-327K, NDUFAF1 peptide 43V-57G or a combination thereof.

25. A method according to claim 24, wherein said binding body comprises an antibody.

26. A method according to claim 25, wherein said antibody is a poly-clonal antibody.

27. A binding body specific for NDUFAF1 peptide 313F-327K, NDUFAF1 peptide 43V-57G or a combination thereof.

28. An antibody according to claim 27.

29. A method for increasing the amount of a complex involved in oxidative phosphorylation in a mitochondrion, comprising providing a cell comprising said mitochondrion with an assembly chaperone for said complex.

30. A method according to claim 29, wherein said complex is complex I.

31. A method according to claim 30, wherein said assembly chaperone comprises NDUFAF1 or a functional part, derivative and/or orthologue thereof.

32. Use of an assembly chaperone for a complex involved in oxidative phosphorylation in a mitochondrion for the preparation of a medicament for the treatment of a condition that is associated with an oxidative phosphorylation deficiency of cells.

33. Use of an assembly chaperone for a complex involved in oxidative phosphorylation in a mitochondrion for increasing the amount of said complex in a mitochondrion of a cell.

34. A method for typing a disease comprising separating complex assembly intermediates in a sample of said individual suffering from said disease and determining relative amounts of two or more of said intermediates and/or altered separation properties of one or more of said complex assembly intermediates.

35. A method according to claim 34, wherein said two or more intermediates are visualized by visualizing the presence in said intermediates of an assembly chaperone.

36. A method according to claim 35, wherein said assembly chaperone comprises NDUFAF1.
